# EUROPEAN PATENT APPLICATION

(11) **EP 3 653 706 A1**
(43) Date of publication of application: **20.05.2020**
(21) Application number: 18205852.9
(22) Date of filing: 13.11.2018
(51) Int. Cl.: C12N 9/42

(54) **FUSION PROTEIN**

(71) Applicant: AB Enzymes Oy, 05200 Rajamäki (FI)
(72) Inventor: JUNTUNEN, Kari, 05200 Rajamäki (FI); VALTAKARI, Leena, 05200 Rajamäki (FI); MÄKINEN, Susanna, 05200 Rajamäki (FI); PURANEN, Terhi, 05200 Rajamäki (FI)
(74) Representative: Espatent Oy

(57) **Abstract**

The invention relates to fusion proteins having depilling, antipilling and/or antigreying performance, and that have improved stability in the presence of proteases. The fusion proteins comprise a cellulase component, a linker component, and a carbohydrate binding component.

SEQUENCE LISTING

<110>  AB Enzymes

<120>  Fusion protein

<130>  31811EP-1f

<160>  21

<170>  PatentIn version 3.5

<210>  1
<211>  38
<212>  PRT
<213>  artificial sequence

<220>
<223>  linker

<400>  1

Val Phe Ser Pro Pro Ser Gly Arg Pro Ser Gly Gly Ser Pro Pro Gly
1               5                   10                  15

Gly Ser Pro Pro Gly Thr Thr Thr Thr Arg Arg Pro Ala Thr Thr Thr
            20                  25                  30

Gly Ser Ser Pro Gly Pro
        35

<210>  2
<211>  38
<212>  PRT
<213>  artificial sequence

<220>
<223>  linker

<400>  2

Val Phe Ser Pro Pro Ser Gly Arg Pro Ser Gly Gly Ser Pro Pro Gly
1               5                   10                  15

Gly Ser Pro Pro Gly Thr Thr Thr Thr Arg Arg Pro Ala Thr Thr Thr
            20                  25                  30

Gly Ser Ser Pro Thr Pro
        35

<210>  3
<211>  38
<212>  PRT
<213>  artificial sequence

<220>
<223>  linker

<400>  3

Val Phe Ser Pro Pro Ser Gly Arg Pro Ser Gly Ser Ser Pro Pro Gly
1               5                   10                  15

Gly Ser Pro Pro Gly Thr Thr Thr Thr Arg Arg Pro Ala Thr Thr Thr
            20                  25                  30

Gly Ser Ser Pro Thr Pro
        35

<210>  4
<211>  298
<212>  PRT
<213>  Acremonium thermophilum

<400>  4

Met Tyr Arg Lys Leu Ala Val Ile Ser Ala Phe Leu Ala Thr Ala Arg
1               5                   10                  15

Ala Leu Asp Gly Lys Ser Thr Arg Tyr Trp Asp Cys Cys Lys Pro Ser
            20                  25                  30

Cys Gly Trp Ala Gly Lys Ala Ser Val Asn Gln Pro Val Phe Ser Cys
        35                  40                  45

Ser Ala Asp Trp Gln Arg Ile Ser Asp Phe Asn Ala Lys Ser Gly Cys
    50                  55                  60

Asp Gly Gly Asn Ala Tyr Ser Cys Ala Asp Gln Thr Pro Trp Ala Val
65                  70                  75                  80

Asn Asp Asn Phe Ser Tyr Gly Phe Ala Ala Thr Ala Ile Ala Gly Gly
                85                  90                  95

Ser Glu Gln Ser Trp Cys Cys Ala Cys Tyr Ala Leu Thr Phe Asn Ser
            100                 105                 110

Gly Pro Val Ala Gly Lys Thr Met Val Val Gln Ser Thr Ser Thr Gly
        115                 120                 125

Gly Asp Leu Gly Glu Asn His Phe Asp Leu Ala Ile Pro Gly Gly Gly
    130                 135                 140

Val Gly Ile Phe Asn Gly Cys Gln Ser Gln Phe Gly Gly Leu Pro Gly
145                 150                 155                 160

Ala Gln Tyr Gly Gly Ile Gln Asp Arg Ser Gln Cys Ser Ser Phe Pro

165                 170                 175

Ala Pro Leu Gln Pro Gly Cys Gln Trp Arg Phe Asp Trp Phe Gln Asn
            180                 185                 190

Ala Asp Asn Pro Thr Phe Thr Phe Gln Arg Val Gln Cys Pro Ser Glu
        195                 200                 205

Leu Thr Ser Arg Thr Gly Cys Lys Arg Asp Asp Asp Ala Asn Tyr Pro
    210                 215                 220

Val Phe Asn Pro Pro Ser Gly Asn Pro Ser Gly Gly Asn Pro Pro Gly
225                 230                 235                 240

Gly Asn Pro Pro Gly Thr Thr Thr Thr Arg Arg Pro Ala Thr Thr Thr
                245                 250                 255

Gly Ser Ser Pro Gly Pro Thr Gln Ser His Tyr Gly Gln Cys Gly Gly
            260                 265                 270

Ile Gly Tyr Ser Gly Pro Thr Val Cys Ala Ser Gly Thr Thr Cys Gln
        275                 280                 285

Val Leu Asn Pro Tyr Tyr Ser Gln Cys Leu
    290                 295

<210>  5
<211>  1079
<212>  DNA
<213>  Acremonium thermophilum

<400>  5
atgtatcgga agttggccgt catctcggcc ttcttggcca cagctcgtgc cctcgacgga      60

aagtcgacga ggtatgccaa tcctcgtacc tctgccctct gtagaaacaa gtgaccgact     120

gcaaagacag atactgggac tgctgcaagc cgtcctgcgg ctgggccgga aaggcctcgg     180

tgaaccagcc cgtcttctcg tgctcggccg actggcagcg catcagcgac ttcaacgcga     240

agtcgggctg cgacggaggc aacgcctact cgtgcgccga ccagacgccc tgggcggtca     300

acgacaactt ctcgtacggc ttcgcagcca cggccatcgc cggcggctcc gagcagagct     360

ggtgctgcgc ctgctatgcg tgagttctct gcaagccgct tcccaccccc gctttctgtg     420

caggccgctt cccccctacc cacccacttc cccccccccg cctctgtgat cgggcatccg     480

agctaagttg cgtgtcgtcc agactcacct tcaactcggg ccccgtcgcg ggcaagacca     540

tggtggtgca gtcgaccagc accggcggcg acctgggcga gaaccacttc gacctcgcca     600

tccccggcgg cggcgtgggc atcttcaacg gctgccagtc ccagttcggc ggcctccccg     660

gcgcccagta cggcggcatc caggaccgca gccagtgctc gtccttcccc gcgccgctcc 720

agccgggctg ccagtggcgc ttcgactggt tccagaacgc cgacaacccc accttcacct 780

tccagcgcgt gcagtgcccg tccgagctca cgtcccgcac gggctgtaag cgcgacgacg 840

acgccaacta tcccgtcttc aacccgcctt cgggcaaccc tagcggcggc aaccctcccg 900

gcggaaaccc gcctggcacc accaccaccc gccgcccagc cactaccact ggaagctctc 960

ccggacctac ccagtctcac tacggccagt gcggcggtat tggctacagc ggccccacgg 1020

tctgcgccag cggcacaact tgccaggtcc tgaaccctta ctactctcag tgcctgtaa 1079

<210> 6
<211> 298
<212> PRT
<213> artificial sequence

<220>
<223> fusion protein

<400> 6

Met Tyr Arg Lys Leu Ala Val Ile Ser Ala Phe Leu Ala Thr Ala Arg
1               5                   10                  15

Ala Leu Asp Gly Lys Ser Thr Arg Tyr Trp Asp Cys Cys Lys Pro Ser
            20                  25                  30

Cys Gly Trp Ala Gly Lys Ala Pro Val Asn Gln Pro Val Phe Ser Cys
        35                  40                  45

Ser Ala Asp Trp Gln Arg Ile Ser Asp Phe Asn Ala Lys Ser Gly Cys
    50                  55                  60

Asp Gly Gly Asn Ala Tyr Ser Cys Ala Asp Gln Thr Pro Trp Ala Val
65                  70                  75                  80

Asn Glu Gln Phe Ser Tyr Gly Phe Ala Ala Thr Ala Ile Ser Gly Gly
                85                  90                  95

Ser Glu Gln Ser Trp Cys Cys Ala Cys Tyr Ala Leu Thr Phe Asn Ser
            100                 105                 110

Gly Pro Val Ala Gly Lys Thr Met Val Val Gln Ser Thr Ser Thr Gly
        115                 120                 125

Gly Asp Leu Gly Glu Asn His Phe Asp Leu Ala Ile Pro Gly Gly Gly
    130                 135                 140

Val Gly Ile Phe Asn Gly Cys Gln Ser Gln Phe Gly Gly Leu Pro Gly
145                 150                 155                 160

Ala Gln Tyr Gly Gly Ile Gln Asp Arg Ser Gln Cys Ser Ser Phe Pro
                165                 170                 175

Ala Pro Leu Gln Pro Gly Cys Tyr Trp Arg Phe Asp Trp Phe Gln Asn
            180                 185                 190

Ala Asp Asn Pro Thr Phe Thr Phe Gln Arg Val Gln Cys Pro Ser Glu
        195                 200                 205

Leu Thr Ser Arg Thr Gly Cys Lys Arg Asp Asp Asp Ala Asn Tyr Pro
    210                 215                 220

Val Phe Ser Pro Pro Ser Gly Arg Pro Ser Gly Gly Ser Pro Pro Gly
225                 230                 235                 240

Gly Ser Pro Pro Gly Thr Thr Thr Thr Arg Arg Pro Ala Thr Thr Thr
                245                 250                 255

Gly Ser Ser Pro Gly Pro Thr Gln Ser His Tyr Gly Gln Cys Gly Gly
            260                 265                 270

Ile Gly Tyr Ser Gly Pro Thr Val Cys Ala Ser Gly Thr Thr Cys Gln
        275                 280                 285

Val Leu Asn Pro Tyr Tyr Ser Gln Cys Leu
    290                 295

<210>  7
<211>  1079
<212>  DNA
<213>  artificial sequence

<220>
<223>  fusion protein

<400>  7
   atgtatcgga agttggccgt catctcggcc ttcttggcca cagctcgtgc cctcgacgga      60
   aagtcgacga ggtatgccaa tcctcgtacc tctgccctct gtagaaacaa gtgaccgact     120
   gcaaagacag atactgggac tgctgcaagc cgtcctgcgg ctgggccgga aaggcccccg     180
   tgaaccagcc cgtcttctcg tgctcggccg actggcagcg catcagcgac ttcaacgcga     240
   agtcgggctg cgacggaggc aacgcctact cgtgcgccga ccagacgccc tgggcggtca     300
   acgagcagtt ctcgtacggc ttcgcagcca cggccatctc cggcggctcc gagcagagct     360
   ggtgctgcgc ctgctatgcg tgagttctct gcaagccgct tcccaccccc gctttctgtg     420
   caggccgctt cccccctacc cacccacttc cccccccccg cctctgtgat cgggcatccg     480
   agctaagttg cgtgtcgtcc agactcacct tcaactcggg ccccgtcgcg ggcaagacca 540
   tggtggtgca gtcgaccagc accggcggcg acctgggcga gaaccacttc gacctcgcca 600
   tccccggcgg cggcgtgggc atcttcaacg gctgccagtc ccagttcggc ggcctccccg 660
   gcgcccagta cggcggcatc caggaccgca gccagtgctc gtccttcccc gcgccgctcc 720
   agccgggctg ctactggcgc ttcgactggt tccagaacgc cgacaacccc accttcacct 780
   tccagcgcgt gcagtgcccg tccgagctca cgtcccgcac gggctgtaag cgcgacgacg 840
   acgccaacta tcccgtcttc tccccgcctt cgggccgccc tagcggcggc tcgcctcccg 900
   gcggatcgcc gcctggcacc accaccaccc gccgcccagc cactaccact ggaagctctc 960
   ccggacctac ccagtctcac tacggccagt gcggcggtat tggctacagc ggccccacgg 1020
   tctgcgccag cggcacaact tgccaggtcc tgaaccctta ctactctcag tgcctgtaa 1079

<210> 8
<211> 298
<212> PRT
<213> artificial sequence

<220>
<223> fusion protein

<400> 8

Met Tyr Arg Lys Leu Ala Val Ile Ser Ala Phe Leu Ala Thr Ala Arg
1               5                   10                  15

Ala Leu Asp Gly Lys Ser Thr Arg Tyr Trp Asp Cys Cys Lys Pro Ser
            20                  25                  30

Cys Gly Trp Ala Gly Lys Ala Pro Val Asn Gln Pro Val Phe Ser Cys
        35                  40                  45

Ser Ala Asp Trp Gln Arg Ile Ser Asp Phe Asn Ala Lys Ser Gly Cys
    50                  55                  60

Asp Gly Gly Asn Ala Tyr Ser Cys Ala Asp Gln Thr Pro Trp Ala Val
65                  70                  75                  80

Asn Glu Gln Phe Ser Tyr Gly Phe Ala Ala Thr Ala Ile Ser Gly Gly
                85                  90                  95

Ser Glu Gln Ser Trp Cys Cys Ala Cys Tyr Ala Leu Thr Phe Asn Ser
            100                 105                 110

Gly Pro Val Ala Gly Lys Thr Met Val Val Gln Ser Thr Ser Thr Gly
        115                 120                 125

Gly Asp Leu Gly Glu Asn His Phe Asp Leu Ala Ile Pro Gly Gly Gly
    130                 135                 140

Val Gly Ile Phe Asn Gly Cys Gln Ser Gln Phe Gly Gly Leu Pro Gly
145                 150                 155                 160

Ala Gln Tyr Gly Gly Ile Gln Asp Arg Ser Gln Cys Ser Ser Phe Pro
                165                 170                 175

Ala Pro Leu Gln Pro Gly Cys Tyr Trp Arg Phe Asp Trp Phe Gln Asn
            180                 185                 190

Ala Asp Asn Pro Thr Phe Thr Phe Gln Arg Val Gln Cys Pro Ser Glu
        195                 200                 205

Leu Thr Ser Arg Thr Gly Cys Lys Arg Asp Asp Asp Ala Asn Tyr Pro
    210                 215                 220

Val Phe Ser Pro Pro Ser Gly Arg Pro Ser Gly Gly Ser Pro Pro Gly
225                 230                 235                 240

Gly Ser Pro Pro Gly Thr Thr Thr Thr Arg Arg Pro Ala Thr Thr Thr
                245                 250                 255

Gly Ser Ser Pro Thr Pro Thr Gln Ser His Tyr Gly Gln Cys Gly Gly
            260                 265                 270

Ile Gly Tyr Ser Gly Pro Thr Val Cys Ala Ser Gly Thr Thr Cys Gln
        275                 280                 285

Val Leu Asn Pro Tyr Tyr Ser Gln Cys Leu
    290                 295

<210>  9
<211>  1079
<212>  DNA
<213>  artificial sequence

<220>
<223>  fusion protein

<400>  9
   atgtatcgga agttggccgt catctcggcc ttcttggcca cagctcgtgc cctcgacgga      60
   aagtcgacga ggtatgccaa tcctcgtacc tctgccctct gtagaaacaa gtgaccgact     120
   gcaaagacag atactgggac tgctgcaagc cgtcctgcgg ctgggccgga aaggcccccg     180
   tgaaccagcc cgtcttctcg tgctcggccg actggcagcg catcagcgac ttcaacgcga     240
   agtcgggctg cgacggaggc aacgcctact cgtgcgccga ccagacgccc tgggcggtca     300
   acgagcagtt ctcgtacggc ttcgcagcca cggccatctc cggcggctcc gagcagagct 360
   ggtgctgcgc ctgctatgcg tgagttctct gcaagccgct tcccaccccc gctttctgtg 420
   caggccgctt cccccctacc cacccacttc cccccccccg cctctgtgat cgggcatccg 480
   agctaagttg cgtgtcgtcc agactcacct tcaactcggg ccccgtcgcg ggcaagacca 540
   tggtggtgca gtcgaccagc accggcggcg acctgggcga gaaccacttc gacctcgcca 600
   tccccggcgg cggcgtgggc atcttcaacg gctgccagtc ccagttcggc ggcctccccg 660
   gcgcccagta cggcggcatc caggaccgca gccagtgctc gtccttcccc gcgccgctcc 720
   agccgggctg ctactggcgc ttcgactggt tccagaacgc cgacaacccc accttcacct 780
   tccagcgcgt gcagtgcccg tccgagctca cgtcccgcac gggctgtaag cgcgacgacg 840
   acgccaacta tcccgtcttc tccccgcctt cgggccgccc tagcggcggc tcgcctcccg 900
   gcggatcgcc gcctggcacc accaccaccc gccgcccagc cactaccact ggaagctctc 960
   ccacccctac ccagtctcac tacggccagt gcggcggtat tggctacagc ggccccacgg 1020
   tctgcgccag cggcacaact tgccaggtcc tgaaccctta ctactctcag tgcctgtaa 1079

<210> 10
<211> 298
<212> PRT
<213> artificial sequence

<220>
<223> fusion protein

<400> 10

Met Tyr Arg Lys Leu Ala Val Ile Ser Ala Phe Leu Ala Thr Ala Arg
1               5                   10                  15

Ala Leu Asp Gly Lys Ser Thr Arg Tyr Trp Asp Cys Cys Lys Pro Ser
            20                  25                  30

Cys Gly Trp Ala Gly Lys Ala Pro Val Asn Gln Pro Val Phe Ser Cys
        35                  40                  45

Ser Ala Asp Trp Gln Arg Ile Ser Asp Phe Asn Ala Lys Ser Gly Cys
    50                  55                  60

Asp Gly Gly Asn Ala Tyr Ser Cys Ala Asp Gln Thr Pro Trp Ala Val
65                  70                  75                  80

Asn Glu Gln Phe Ser Tyr Gly Phe Ala Ala Thr Ala Ile Ser Gly Gly
                85                  90                  95

Ser Glu Gln Ser Trp Cys Cys Ala Cys Tyr Ala Leu Thr Phe Asn Ser
            100                 105                 110

Gly Pro Val Ala Gly Lys Thr Met Val Val Gln Ser Thr Ser Thr Gly
        115                 120                 125

Gly Asp Leu Gly Glu Asn His Phe Asp Leu Ala Ile Pro Gly Gly Gly
    130                 135                 140

Val Gly Ile Phe Asn Gly Cys Gln Ser Gln Phe Gly Gly Leu Pro Gly
145                 150                 155                 160

Ala Gln Tyr Gly Gly Ile Gln Asp Arg Ser Gln Cys Ser Ser Phe Pro
                165                 170                 175

Ala Pro Leu Gln Pro Gly Cys Tyr Trp Arg Phe Asp Trp Phe Gln Asn
            180                 185                 190

Ala Asp Asn Pro Thr Phe Thr Phe Gln Arg Val Gln Cys Pro Ser Glu
        195                 200                 205

Leu Thr Ser Arg Thr Gly Cys Lys Arg Asp Asp Asp Ala Asn Tyr Pro
    210                 215                 220

Val Phe Ser Pro Pro Ser Gly Arg Pro Ser Gly Ser Ser Pro Pro Gly
225                 230                 235                 240

Gly Ser Pro Pro Gly Thr Thr Thr Thr Arg Arg Pro Ala Thr Thr Thr
                245                 250                 255

Gly Ser Ser Pro Thr Pro Thr Gln Ser His Tyr Gly Gln Cys Gly Gly
            260                 265                 270

Ile Gly Tyr Ser Gly Pro Thr Val Cys Ala Ser Gly Thr Thr Cys Gln
        275                 280                 285

Val Leu Asn Pro Tyr Tyr Ser Gln Cys Leu
    290                 295

<210>  11
<211>  1079
<212>  DNA
<213>  artificial sequence

<220>
<223>  fusion protein

<400>  11
atgtatcgga agttggccgt catctcggcc ttcttggcca cagctcgtgc cctcgacgga        60

aagtcgacga ggtatgccaa tcctcgtacc tctgccctct gtagaaacaa gtgaccgact       120

gcaaagacag atactgggac tgctgcaagc cgtcctgcgg ctgggccgga aaggcccccg 180

tgaaccagcc cgtcttctcg tgctcggccg actggcagcg catcagcgac ttcaacgcga 240

agtcgggctg cgacggaggc aacgcctact cgtgcgccga ccagacgccc tgggcggtca 300

acgagcagtt ctcgtacggc ttcgcagcca cggccatctc cggcggctcc gagcagagct 360

ggtgctgcgc ctgctatgcg tgagttctct gcaagccgct tcccaccccc gctttctgtg 420

caggccgctt cccccctacc cacccacttc cccccccccg cctctgtgat cgggcatccg 480

agctaagttg cgtgtcgtcc agactcacct tcaactcggg ccccgtcgcg ggcaagacca 540

tggtggtgca gtcgaccagc accggcggcg acctgggcga gaaccacttc gacctcgcca 600

tccccggcgg cggcgtgggc atcttcaacg gctgccagtc ccagttcggc ggcctccccg 660

gcgcccagta cggcggcatc caggaccgca gccagtgctc gtccttcccc gcgccgctcc 720

agccgggctg ctactggcgc ttcgactggt tccagaacgc cgacaacccc accttcacct 780

tccagcgcgt gcagtgcccg tccgagctca cgtcccgcac gggctgtaag cgcgacgacg 840

acgccaacta tcccgtcttc tccccgcctt cgggccgccc tagcggctcg tcgcctcccg 900

gcggatcgcc gcctggcacc accaccaccc gccgcccagc cactaccact ggaagctctc 960

ccacccctac ccagtctcac tacggccagt gcggcggtat tggctacagc ggccccacgg 1020

tctgcgccag cggcacaact tgccaggtcc tgaaccctta ctactctcag tgcctgtaa 1079

<210> 12
<211> 235
<212> PRT
<213> Melanocarpus albomyces

<400> 12

Met Arg Ser Thr Pro Val Leu Arg Ala Leu Leu Ala Ala Ala Leu Pro
1               5                   10                  15

Leu Gly Ala Leu Ala Ala Asn Gly Gln Ser Thr Arg Tyr Trp Asp Cys
            20                  25                  30

Cys Lys Pro Ser Cys Gly Trp Ala Gly Lys Ala Pro Val Asn Gln Pro
        35                  40                  45

Val Tyr Ser Cys Asp Ala Asn Phe Gln Arg Ile His Asp Phe Asp Ala
    50                  55                  60

Val Ser Gly Cys Glu Gly Gly Pro Ala Phe Ser Cys Ala Asp His Ser
65                  70                  75                  80

Pro Trp Ala Ile Asn Asp Asn Leu Ser Tyr Gly Phe Ala Ala Thr Ala
                85                  90                  95

Leu Ser Gly Gln Thr Glu Glu Ser Trp Cys Cys Ala Cys Tyr Ala Leu
            100                 105                 110

Thr Phe Thr Ser Gly Pro Val Ala Gly Lys Thr Met Val Val Gln Ser
        115                 120                 125

Thr Ser Thr Gly Gly Asp Leu Gly Ser Asn His Phe Asp Leu Ala Ile
    130                 135                 140

Pro Gly Gly Gly Val Gly Leu Phe Asp Gly Cys Thr Pro Gln Phe Gly
145                 150                 155                 160

Gly Leu Pro Gly Ala Arg Tyr Gly Gly Ile Ser Ser Arg Gln Glu Cys
                165                 170                 175

Asp Ser Phe Pro Glu Pro Leu Lys Pro Gly Cys Gln Trp Arg Phe Asp
            180                 185                 190

Trp Phe Gln Asn Ala Asp Asn Pro Ser Phe Thr Phe Glu Arg Val Gln
        195                 200                 205

Cys Pro Glu Glu Leu Val Ala Arg Thr Gly Cys Arg Arg His Asp Asp
    210                 215                 220

Gly Gly Phe Ala Val Phe Lys Ala Pro Ser Ala
225                 230                 235

<210>  13
<211>  849
<212>  DNA
<213>  Melanocarpus albomyces

<400>  13
atgcgctcta ctcccgttct ccgcgccctc ctggccgcag cattgcccct cggggccctc      60

gccgccaacg gtcagtccac gaggtaactg atcacccgcc tcattacgcg tgccgaccgg     120

accgcgttca gggctcactg ctcaccgcat ccagatactg ggactgctgc aagccgtcgt     180

gcggctgggc cggaaaggcc cccgtgaacc agcccgtcta ctcgtgcgac gccaacttcc     240

agcgcatcca cgacttcgat gccgtctcgg gctgcgaggg cggccccgcc ttctcgtgcg     300

ccgaccacag cccctgggcc attaatgaca acctctcgta cggcttcgcg gcgactgcac     360

tcagcggcca gaccgaggag tcgtggtgct gtgcctgcta cgcgtgagtg tgcttgggcc     420

caacgtcggt gattccggag ttcagaccac tgacccagcg acccgctcgc cagtctgacc     480

tttacatcgg gtcccgtggc cggcaagacc atggtcgtcc agtcgaccag cacgggcggc     540

gacctcggca gcaaccactt cgacctcgcc atccccggcg gcggcgtcgg cctcttcgac     600

ggctgcactc cccagttcgg cggcctcccg ggcgcacggt acggcggcat ctcgtcgcgc 660

caggagtgcg actcgttccc cgagccgctc aagcccggct gccagtggcg cttcgactgg 720

ttccagaacg ccgacaaccc gtcctttacc ttcgagcggg tccagtgccc cgaggagctg 780

gtcgctcgga ccggctgcag gcgccacgac gacggcggct tcgccgtctt caaggccccc 840

agcgcctga 849

<210> 14
<211> 304
<212> PRT
<213> artificial sequence

<220>
<223> fusion protein

<400> 14

Met Arg Ser Thr Pro Val Leu Arg Ala Leu Leu Ala Ala Ala Leu Pro
1               5                   10                  15

Leu Gly Ala Leu Ala Ala Asn Gly Gln Ser Thr Arg Tyr Trp Asp Cys
            20                  25                  30

Cys Lys Pro Ser Cys Gly Trp Ala Gly Lys Ala Pro Val Asn Gln Pro
        35                  40                  45

Val Tyr Ser Cys Asp Ala Asn Phe Gln Arg Ile His Asp Phe Asp Ala
    50                  55                  60

Val Ser Gly Cys Glu Gly Gly Pro Ala Phe Ser Cys Ala Asp His Ser
65                  70                  75                  80

Pro Trp Ala Ile Asn Asp Asn Leu Ser Tyr Gly Phe Ala Ala Thr Ala
                85                  90                  95

Leu Ser Gly Gln Thr Glu Glu Ser Trp Cys Cys Ala Cys Tyr Ala Leu
            100                 105                 110

Thr Phe Thr Ser Gly Pro Val Ala Gly Lys Thr Met Val Val Gln Ser
        115                 120                 125

Thr Ser Thr Gly Gly Asp Leu Gly Ser Asn His Phe Asp Leu Ala Ile
    130                 135                 140

Pro Gly Gly Gly Val Gly Leu Phe Asp Gly Cys Thr Pro Gln Phe Gly
145                 150                 155                 160

Gly Leu Pro Gly Ala Arg Tyr Gly Gly Ile Ser Ser Arg Gln Glu Cys
                165                 170                 175

Asp Ser Phe Pro Glu Pro Leu Lys Pro Gly Cys Gln Trp Arg Phe Asp
            180                 185                 190

Trp Phe Gln Asn Ala Asp Asn Pro Ser Phe Thr Phe Glu Arg Val Gln
        195                 200                 205

Cys Pro Glu Glu Leu Val Ala Arg Thr Gly Cys Arg Arg His Asp Asp
    210                 215                 220

Gly Gly Phe Pro Ala Val Gln Ile Pro Ser Ser Thr Gly Asn Pro Ser
225                 230                 235                 240

Gly Gly Asn Pro Pro Gly Gly Asn Pro Pro Gly Thr Thr Thr Thr Arg
                245                 250                 255

Arg Pro Ala Thr Thr Thr Gly Ser Ser Pro Gly Pro Thr Gln Ser His
            260                 265                 270

Tyr Gly Gln Cys Gly Gly Ile Gly Tyr Ser Gly Pro Thr Val Cys Ala
        275                 280                 285

Ser Gly Thr Thr Cys Gln Val Leu Asn Pro Tyr Tyr Ser Gln Cys Leu
    290                 295                 300

<210>  15
<211>  1056
<212>  DNA
<213>  artificial sequence

<220>
<223>  fusion protein

<400>  15
atgcgctcta ctcccgttct ccgcgccctc ctggccgcag cattgcccct cggggccctc      60

gccgccaacg gtcagtccac gaggtaactg atcacccgcc tcattacgcg tgccgaccgg     120

accgcgttca gggctcactg ctcaccgcat ccagatactg ggactgctgc aagccgtcgt     180

gcggctgggc cggaaaggcc cccgtgaacc agcccgtcta ctcgtgcgac gccaacttcc     240

agcgcatcca cgacttcgat gccgtctcgg gctgcgaggg cggccccgcc ttctcgtgcg     300

ccgaccacag cccctgggcc attaatgaca acctctcgta cggcttcgcg gcgactgcac     360

tcagcggcca gaccgaggag tcgtggtgct gtgcctgcta cgcgtgagtg tgcttgggcc     420

caacgtcggt gattccggag ttcagaccac tgacccagcg acccgctcgc cagtctgacc     480

tttacatcgg gtcccgtggc cggcaagacc atggtcgtcc agtcgaccag cacgggcggc     540

gacctcggca gcaaccactt cgacctcgcc atccccggcg gcggcgtcgg cctcttcgac     600

ggctgcactc cccagttcgg cggcctcccg ggcgcacggt acggcggcat ctcgtcgcgc 660

caggagtgcg actcgttccc cgagccgctc aagcccggct gccagtggcg cttcgactgg 720

ttccagaacg ccgacaaccc gtcctttacc ttcgagcggg tccagtgccc cgaggagctg 780

gtcgctcgga ccggctgcag gcgccacgac gacggcggct tccccgccgt ccagatcccc 840

tcgtcgaccg gcaaccctag cggcggcaac cctcccggcg gaaacccgcc tggcaccacc 900

accacccgcc gcccagccac taccactgga agctctcccg gacctaccca gtctcactac 960

ggccagtgcg gcggtattgg ctacagcggc cccacggtct gcgccagcgg cacaacttgc 1020

caggtcctga acccttacta ctctcagtgc ctgtaa 1056

<210> 16
<211> 302
<212> PRT
<213> artificial sequence

<220>
<223> fusion protein

<400> 16

Met Arg Ser Thr Pro Val Leu Arg Ala Leu Leu Ala Ala Ala Leu Pro
1               5                   10                  15

Leu Gly Ala Leu Ala Ala Asn Gly Gln Ser Thr Arg Tyr Trp Asp Cys
            20                  25                  30

Cys Lys Pro Ser Cys Gly Trp Ala Gly Lys Ala Pro Val Asn Gln Pro
        35                  40                  45

Val Tyr Ser Cys Asp Ala Asn Phe Gln Arg Ile His Asp Phe Asp Ala
    50                  55                  60

Val Ser Gly Cys Glu Gly Gly Pro Ala Phe Ser Cys Ala Asp His Ser
65                  70                  75                  80

Pro Trp Ala Ile Asn Asp Asn Leu Ser Tyr Gly Phe Ala Ala Thr Ala
                85                  90                  95

Leu Ser Gly Gln Thr Glu Glu Ser Trp Cys Cys Ala Cys Tyr Ala Leu
            100                 105                 110

Thr Phe Thr Ser Gly Pro Val Ala Gly Lys Thr Met Val Val Gln Ser
        115                 120                 125

Thr Ser Thr Gly Gly Asp Leu Gly Ser Asn His Phe Asp Leu Ala Ile
    130                 135                 140

Pro Gly Gly Gly Val Gly Leu Phe Asp Gly Cys Thr Pro Gln Phe Gly
145                 150                 155                 160

Gly Leu Pro Gly Ala Arg Tyr Gly Gly Ile Ser Ser Arg Gln Glu Cys
                165                 170                 175

Asp Ser Phe Pro Glu Pro Leu Lys Pro Gly Cys Gln Trp Arg Phe Asp
            180                 185                 190

Trp Phe Gln Asn Ala Asp Asn Pro Ser Phe Thr Phe Glu Arg Val Gln
        195                 200                 205

Cys Pro Glu Glu Leu Val Ala Arg Thr Gly Cys Arg Arg His Asp Asp
    210                 215                 220

Gly Gly Phe Pro Val Phe Ser Pro Pro Ser Gly Arg Pro Ser Gly Gly
225                 230                 235                 240

Ser Pro Pro Gly Gly Ser Pro Pro Gly Thr Thr Thr Thr Arg Arg Pro
                245                 250                 255

Ala Thr Thr Thr Gly Ser Ser Pro Gly Pro Thr Gln Ser His Tyr Gly
            260                 265                 270

Gln Cys Gly Gly Ile Gly Tyr Ser Gly Pro Thr Val Cys Ala Ser Gly
        275                 280                 285

Thr Thr Cys Gln Val Leu Asn Pro Tyr Tyr Ser Gln Cys Leu
    290                 295                 300

<210>  17
<211>  1050
<212>  DNA
<213>  artificial sequence

<220>
<223>  fusion protein

<400>  17
atgcgctcta ctcccgttct ccgcgccctc ctggccgcag cattgcccct cggggccctc      60

gccgccaacg gtcagtccac gaggtaactg atcacccgcc tcattacgcg tgccgaccgg     120

accgcgttca gggctcactg ctcaccgcat ccagatactg ggactgctgc aagccgtcgt     180

gcggctgggc cggaaaggcc cccgtgaacc agcccgtcta ctcgtgcgac gccaacttcc     240

agcgcatcca cgacttcgat gccgtctcgg gctgcgaggg cggccccgcc ttctcgtgcg     300

ccgaccacag cccctgggcc attaatgaca acctctcgta cggcttcgcg gcgactgcac     360

tcagcggcca gaccgaggag tcgtggtgct gtgcctgcta cgcgtgagtg tgcttgggcc     420

caacgtcggt gattccggag ttcagaccac tgacccagcg acccgctcgc cagtctgacc 480

tttacatcgg gtcccgtggc cggcaagacc atggtcgtcc agtcgaccag cacgggcggc 540

gacctcggca gcaaccactt cgacctcgcc atccccggcg gcggcgtcgg cctcttcgac 600

ggctgcactc cccagttcgg cggcctcccg ggcgcacggt acggcggcat ctcgtcgcgc 660

caggagtgcg actcgttccc cgagccgctc aagcccggct gccagtggcg cttcgactgg 720

ttccagaacg ccgacaaccc gtcctttacc ttcgagcggg tccagtgccc cgaggagctg 780

gtcgctcgga ccggctgcag gcgccacgac gacggcggct tccccgtctt ctccccgcct 840

tcgggccgcc ctagcggcgg ctcgcctccc ggcggatcgc cgcctggcac caccaccacc 900

cgccgcccag ccactaccac tggaagctct cccggaccta cccagtctca ctacggccag 960

tgcggcggta ttggctacag cggccccacg gtctgcgcca gcggcacaac ttgccaggtc 1020

ctgaaccctt actactctca gtgcctgtaa 1050

<210> 18
<211> 302
<212> PRT
<213> atificial sequence

<400> 18

Met Arg Ser Thr Pro Val Leu Arg Ala Leu Leu Ala Ala Ala Leu Pro
1               5                   10                  15

Leu Gly Ala Leu Ala Ala Asn Gly Gln Ser Thr Arg Tyr Trp Asp Cys
            20                  25                  30

Cys Lys Pro Ser Cys Gly Trp Ala Gly Lys Ala Pro Val Asn Gln Pro
        35                  40                  45

Val Tyr Ser Cys Asp Ala Asn Phe Gln Arg Ile His Asp Phe Asp Ala
    50                  55                  60

Val Ser Gly Cys Glu Gly Gly Pro Ala Phe Ser Cys Ala Asp His Ser
65                  70                  75                  80

Pro Trp Ala Ile Asn Asp Asn Leu Ser Tyr Gly Phe Ala Ala Thr Ala
                85                  90                  95

Leu Ser Gly Gln Thr Glu Glu Ser Trp Cys Cys Ala Cys Tyr Ala Leu
            100                 105                 110

Thr Phe Thr Ser Gly Pro Val Ala Gly Lys Thr Met Val Val Gln Ser
        115                 120                 125

Thr Ser Thr Gly Gly Asp Leu Gly Ser Asn His Phe Asp Leu Ala Ile

130                 135                 140

Pro Gly Gly Gly Val Gly Leu Phe Asp Gly Cys Thr Pro Gln Phe Gly
145                 150                 155                 160

Gly Leu Pro Gly Ala Arg Tyr Gly Gly Ile Ser Ser Arg Gln Glu Cys
                165                 170                 175

Asp Ser Phe Pro Glu Pro Leu Lys Pro Gly Cys Gln Trp Arg Phe Asp
            180                 185                 190

Trp Phe Gln Asn Ala Asp Asn Pro Ser Phe Thr Phe Glu Arg Val Gln
        195                 200                 205

Cys Pro Glu Glu Leu Val Ala Arg Thr Gly Cys Arg Arg His Asp Asp
    210                 215                 220

Gly Gly Phe Pro Val Phe Ser Pro Pro Ser Gly Arg Pro Ser Gly Gly
225                 230                 235                 240

Ser Pro Pro Gly Gly Ser Pro Pro Gly Thr Thr Thr Thr Arg Arg Pro
                245                 250                 255

Ala Thr Thr Thr Gly Ser Ser Pro Thr Pro Thr Gln Ser His Tyr Gly
            260                 265                 270

Gln Cys Gly Gly Ile Gly Tyr Ser Gly Pro Thr Val Cys Ala Ser Gly
        275                 280                 285

Thr Thr Cys Gln Val Leu Asn Pro Tyr Tyr Ser Gln Cys Leu
    290                 295                 300

<210>  19
<211>  1050
<212>  DNA
<213>  artificial sequence

<220>
<223>  fusion protein

<400>  19
atgcgctcta ctcccgttct ccgcgccctc ctggccgcag cattgcccct cggggccctc      60

gccgccaacg gtcagtccac gaggtaactg atcacccgcc tcattacgcg tgccgaccgg     120

accgcgttca gggctcactg ctcaccgcat ccagatactg ggactgctgc aagccgtcgt     180

gcggctgggc cggaaaggcc cccgtgaacc agcccgtcta ctcgtgcgac gccaacttcc     240

agcgcatcca cgacttcgat gccgtctcgg gctgcgaggg cggccccgcc ttctcgtgcg     300

ccgaccacag cccctgggcc attaatgaca acctctcgta cggcttcgcg gcgactgcac     360

tcagcggcca gaccgaggag tcgtggtgct gtgcctgcta cgcgtgagtg tgcttgggcc 420

caacgtcggt gattccggag ttcagaccac tgacccagcg acccgctcgc cagtctgacc 480

tttacatcgg gtcccgtggc cggcaagacc atggtcgtcc agtcgaccag cacgggcggc 540

gacctcggca gcaaccactt cgacctcgcc atccccggcg gcggcgtcgg cctcttcgac 600

ggctgcactc cccagttcgg cggcctcccg ggcgcacggt acggcggcat ctcgtcgcgc 660

caggagtgcg actcgttccc cgagccgctc aagcccggct gccagtggcg cttcgactgg 720

ttccagaacg ccgacaaccc gtcctttacc ttcgagcggg tccagtgccc cgaggagctg 780

gtcgctcgga ccggctgcag gcgccacgac gacggcggct tccccgtctt ctccccgcct 840

tcgggccgcc ctagcggcgg ctcgcctccc ggcggatcgc cgcctggcac caccaccacc 900

cgccgcccag ccactaccac tggaagctct cccaccccta cccagtctca ctacggccag 960

tgcggcggta ttggctacag cggccccacg gtctgcgcca gcggcacaac ttgccaggtc 1020

ctgaaccctt actactctca gtgcctgtaa 1050

<210> 20
<211> 302
<212> PRT
<213> artificial sequence

<220>
<223> fusion protein

<400> 20

Met Arg Ser Thr Pro Val Leu Arg Ala Leu Leu Ala Ala Ala Leu Pro
1               5                   10                  15

Leu Gly Ala Leu Ala Ala Asn Gly Gln Ser Thr Arg Tyr Trp Asp Cys
            20                  25                  30

Cys Lys Pro Ser Cys Gly Trp Ala Gly Lys Ala Pro Val Asn Gln Pro
        35                  40                  45

Val Tyr Ser Cys Asp Ala Asn Phe Gln Arg Ile His Asp Phe Asp Ala
    50                  55                  60

Val Ser Gly Cys Glu Gly Gly Pro Ala Phe Ser Cys Ala Asp His Ser
65                  70                  75                  80

Pro Trp Ala Ile Asn Asp Asn Leu Ser Tyr Gly Phe Ala Ala Thr Ala
                85                  90                  95

Leu Ser Gly Gln Thr Glu Glu Ser Trp Cys Cys Ala Cys Tyr Ala Leu
            100                 105                 110

Thr Phe Thr Ser Gly Pro Val Ala Gly Lys Thr Met Val Val Gln Ser
        115                 120                 125

Thr Ser Thr Gly Gly Asp Leu Gly Ser Asn His Phe Asp Leu Ala Ile
    130                 135                 140

Pro Gly Gly Gly Val Gly Leu Phe Asp Gly Cys Thr Pro Gln Phe Gly
145                 150                 155                 160

Gly Leu Pro Gly Ala Arg Tyr Gly Gly Ile Ser Ser Arg Gln Glu Cys
                165                 170                 175

Asp Ser Phe Pro Glu Pro Leu Lys Pro Gly Cys Gln Trp Arg Phe Asp
            180                 185                 190

Trp Phe Gln Asn Ala Asp Asn Pro Ser Phe Thr Phe Glu Arg Val Gln
        195                 200                 205

Cys Pro Glu Glu Leu Val Ala Arg Thr Gly Cys Arg Arg His Asp Asp
    210                 215                 220

Gly Gly Phe Pro Val Phe Ser Pro Pro Ser Gly Arg Pro Ser Gly Ser
225                 230                 235                 240

Ser Pro Pro Gly Gly Ser Pro Pro Gly Thr Thr Thr Thr Arg Arg Pro
                245                 250                 255

Ala Thr Thr Thr Gly Ser Ser Pro Thr Pro Thr Gln Ser His Tyr Gly
            260                 265                 270

Gln Cys Gly Gly Ile Gly Tyr Ser Gly Pro Thr Val Cys Ala Ser Gly
        275                 280                 285

Thr Thr Cys Gln Val Leu Asn Pro Tyr Tyr Ser Gln Cys Leu
    290                 295                 300

<210>  21
<211>  1050
<212>  DNA
<213>  artificial sequence

<220>
<223>  fusion protein

<400>  21
atgcgctcta ctcccgttct ccgcgccctc ctggccgcag cattgcccct cggggccctc      60

gccgccaacg gtcagtccac gaggtaactg atcacccgcc tcattacgcg tgccgaccgg     120

accgcgttca gggctcactg ctcaccgcat ccagatactg ggactgctgc aagccgtcgt     180

gcggctgggc cggaaaggcc cccgtgaacc agcccgtcta ctcgtgcgac gccaacttcc 240

agcgcatcca cgacttcgat gccgtctcgg gctgcgaggg cggccccgcc ttctcgtgcg 300

ccgaccacag cccctgggcc attaatgaca acctctcgta cggcttcgcg gcgactgcac 360

tcagcggcca gaccgaggag tcgtggtgct gtgcctgcta cgcgtgagtg tgcttgggcc 420

caacgtcggt gattccggag ttcagaccac tgacccagcg acccgctcgc cagtctgacc 480

tttacatcgg gtcccgtggc cggcaagacc atggtcgtcc agtcgaccag cacgggcggc 540

gacctcggca gcaaccactt cgacctcgcc atccccggcg gcggcgtcgg cctcttcgac 600

ggctgcactc cccagttcgg cggcctcccg ggcgcacggt acggcggcat ctcgtcgcgc 660

caggagtgcg actcgttccc cgagccgctc aagcccggct gccagtggcg cttcgactgg 720

ttccagaacg ccgacaaccc gtcctttacc ttcgagcggg tccagtgccc cgaggagctg 780

gtcgctcgga ccggctgcag gcgccacgac gacggcggct tccccgtctt ctccccgcct 840

tcgggccgcc ctagcggctc gtcgcctccc ggcggatcgc cgcctggcac caccaccacc 900

cgccgcccag ccactaccac tggaagctct cccaccccta cccagtctca ctacggccag 960

tgcggcggta ttggctacag cggccccacg gtctgcgcca gcggcacaac ttgccaggtc 1020

ctgaaccctt actactctca gtgcctgtaa 1050

## Description

### FIELD OF THE INVENTION

The present invention relates to fusion proteins having cellulase activity and having a capability to bind carbohydrate, as well as to methods for their production, products containing such fusion proteins, and to use of the fusion proteins in various industrial applications.

### BACKGROUND

Cellulose is a linear polysaccharide of glucose residues connected by β-1,4 linkages. Cellulose is the main component of plant cell walls, and the basic building block of many textiles and paper. It gives plant cells remarkable strength helping them to resist mechanical stress and osmotic pressure. Cotton is the purest natural form of cellulose.

Cellulases or cellulolytic enzymes are a group of glycoside hydrolase enzymes that catalyze the hydrolysis of beta-1,4 glycosidic linkages in the cellulose polymer. Cellulases are known to be produced by a large number of bacteria, yeast, and fungi. Cellulases comprise a catalytic domain/core (CD) expressing cellulase activity. In addition to the catalytic domain, some cellulase molecules may comprise one or more cellulose binding domains (CBDs), also named as carbohydrate binding domains/modules (CBD/CBM).

Cellulases or cellulolytic enzymes are enzymes involved in hydrolysis of cellulose. In the textile industry, cellulases are used in denim finishing to create a fashionable stone washed appearance in denim cloths in a biostoning process, and they are also used, for instance, to clean fuzz and prevent formation of pills on the surface of cotton garments. In detergent industry cellulases are used to brighten colors and to prevent graying, pilling of garments and to improve cleaning. Cellulases are further used in food industry and animal feed manufacturing, and they have a great potential in biomass hydrolysis and in the pulp and paper industry, for instance, in deinking to release ink from fiber surfaces and in improving pulp drainage. Even though some commercial cellulase products have been brought to the market, there remains a need for cellulases that are less sensitive for proteolytic degradation and have improved stability and performance in varying industrial applications.

### SUMMARY OF THE INVENTION

An object of the invention is to provide novel enzymes that can be used to prevent and remove pilling from fabrics, and that have improved stability in liquid detergents containing proteases. The inventors of the present invention achieved this by developing fusion proteins that contain a cellulase component, a linker component and a carbohydrate binding component, each being engineered to have a sequence which contributes to the properties of the fusion protein and makes it suitable for industrial production and use. The present fusion protein has depilling and antipilling effect, which cannot be achieved with the catalytic core of the cellulase alone, or with cellulases without a carbohydrate binding domain. This fusion protein is efficient also in antigreying.

According to the first aspect of the invention is provided a fusion protein comprising a cellulase component, a linker component, and a carbohydrate binding component, wherein:
- the cellulase component is an endoglucanase;
- the linker component has at least 90% sequence identity with SEQ ID NO: 1, 2 or 3; and
- the carbohydrate binding component has capability to bind cellulose.

According to the second aspect of the invention is provided an isolated nucleic acid encoding the fusion protein of the first aspect.

According to the third aspect of the invention is provided a recombinant expression vector comprising the isolated nucleic acid of the second aspect operably linked to regulatory sequences capable of directing expression of a gene encoding said fusion cellulase in a host.

According to the fourth aspect of the invention is provided a host cell comprising the fusion protein of the first aspect, or the recombinant expression vector of the third aspect.

According to the fifth aspect of the invention is provided a method of producing the fusion protein of the first aspect comprising the steps of transforming a host cell with an expression vector encoding said fusion protein, culturing said host cell under conditions enabling expression of said fusion protein, and optionally recovering and purifying said fusion protein.

According to the sixth aspect of the invention is provided an enzyme composition comprising the fusion protein of the first aspect.

According to the seventh aspect of the invention is provided a detergent composition comprising the fusion protein of the first aspect or an enzyme composition of the sixth aspect.

According to the eighth aspect of the invention is provided a method for treating cellulosic material, wherein the method comprises reacting the cellulosic material with the fusion protein of the first aspect or the enzyme composition of the sixth aspect.

According to the ninth aspect of the invention is provided a use of the fusion protein of the first aspect, or the enzyme composition of the sixth aspect in textile and detergent industry, in biomass processing, in biofuel, starch, pulp and paper, food, baking, feed or beverage industry.

According to another aspect is provided a method for antigreying, stain removal, fiber and color care, biostoning or biofinishing, which comprises a step of adding the present fusion protein or the present enzyme preparation to liquid used in treating fabric containing cellulose or cellulose derivate or garments or other textile materials like fabrics or garments or yarn.

### FIGURES

Figure 1A shows a schematic picture of the expression plasmid used in the transformation of *Trichoderma reesei* for expression of the gene of interest (GOI). The recombinant genes were under control of *T. reesei cel7*A promoter (Pcel7A) and transcription termination was ensured with the addition of the *T. reesei cel7*A (Tcel7A) terminator. The amdS gene (amdS) was included for selection of the transformants. Ampicillin resistance gene (bla) was used in plasmid construction. Picture was generated using Geneious version 11.0 created by Biomatters.

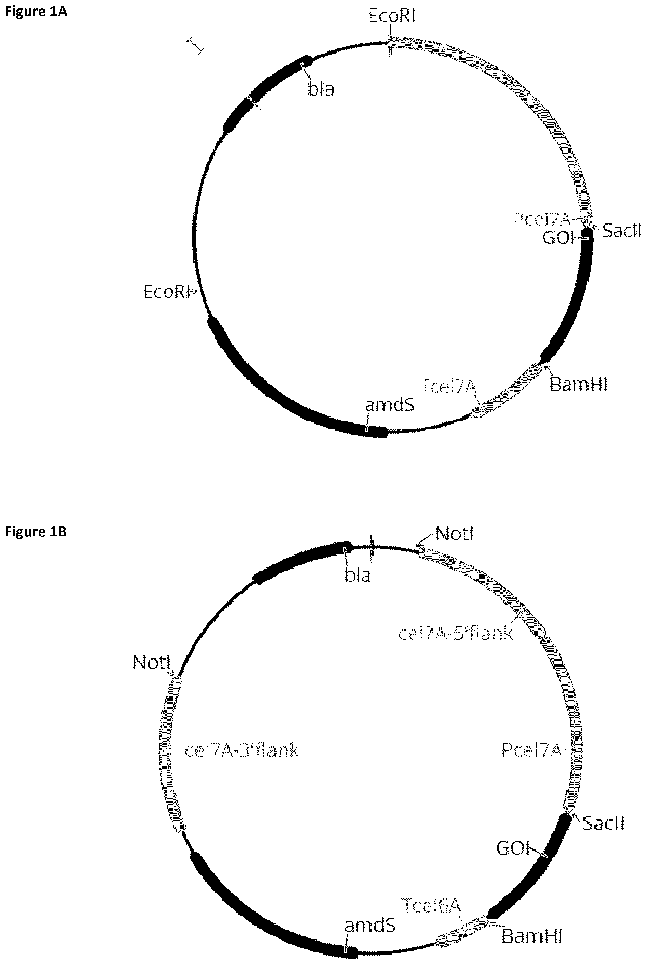

Figure 1B shows a schematic picture of the expression plasmid used in the transformation of *Trichoderma reesei* for expression of the gene of interest (GOI). The recombinant genes were under control of *T. reesei cel7*A promoter (Pcel7A) and transcription termination was ensured with the addition of *T. reesei cel6*A (Tcel6A) terminator. Upstream and downstream regions from cel7A were included in the plasmid (flanks). The amdS gene (amdS) was included for selection of the transformants. Ampicillin resistance gene (bla) was used in plasmid construction. Picture was generated using Geneious version 11.0 created by Biomatters.
Figure 2 shows the color revival (pilling removal/depilling) performance of the fusion proteins MA79+CBM and MA79+CBML compared to MA79 as an increase of darkness (sum of -ΔL*of 4 stripes) after 10 washing and tumbling cycles of prepilled test monitors (E-253). Washing conditions in Launder-Ometer were: 40°C, 60 min, 16°dH, detergent 4.4 g/l, pH ∼8.4, enzyme dosage 0 - 0.2 NCU/ml.
Figure 3 shows the color care (antipilling) performance of MA79+CBM and MA79+CBML compared to MA79 as an increase of darkness (sum of -ΔL*of 4 stripes) after 10 washing and tumbling cycles of unwashed, new test monitors (E-252). Washing conditions in Launder-Ometer were: 40°C, 60 min, 16°dH, detergent 4.4 g/l, pH ∼8.4, enzyme dosage 0 - 0.2 NCU/ml.
Figure 4 shows the residual performance (%) of fusion protein MA79+CBML compared to MA79+CBM as color revival (depilling) effect after storage in commercial liquid detergent containing protease (0.7 % Savinase 16 L) at room temperature (approx. 20 - 22°C) for 4 days.
Figure 5 shows the residual performance (%) of fusion proteins DC144, DC145 and DC146 compared to ACM88 as color revival (depilling) effect after storage in commercial liquid detergent containing protease (0.7 % Savinase 16 L) at room temperature (approx. 20 - 22°C) for 4 days.
Figure 6 shows the residual NCU activity (%) of fusion proteins DC144, DC145 and DC146 compared to ACM88 effect after storage in commercial liquid detergent containing protease (0.7 % Savinase 16 L) at room temperature (approx. 20 - 22°C) for 4 days.
Figure 7 shows the color revival (pilling removal/depilling) performance of the fusion proteins DC144, DC145 and DC146 compared to ACM88 as an increase of darkness (sum of -ΔL*of 4 stripes) after 3 washing and tumbling cycles of prepilled test monitors (E-253). Washing conditions in Launder-Ometer were: 40°C, 60 min, 16°dH, detergent 4.4 g/l, pH -8.4, enzyme dosage 2 NCU/ml.
Figure 8 shows the residual performance (%) of fusion proteins DC144, DC145 and DC146 compared to ACM88 as color revival (depilling) effect after storage (30°C, 11 d) in commercial liquid detergent concentrate containing protease.
Figure 9 shows the residual performance (%) of fusion protein MA79+CBML compared to MA79+CBM as color revival (depilling) effect after storage (30°C, 11 d) in commercial liquid detergent concentrate containing protease.
Figure 10 shows the antigreying performance of fusion proteins MA79+CBM and MA79+CBML compared to MA79 in a single wash in the presence of carbon black in a commercial liquid detergent. Washing conditions: 40°C, 60 min, 16°dH, carbon black approx. 0.15 g/l, detergent 4.4 g/l, enzyme dosage 0 - 0.2 NCU/ml.
Figure 11 shows the residual performance (%) of fusion protein MA79+CBML compared to MA79 as antigreying effect after storage (37°C, 11 d) in commercial liquid detergent containing protease (0.8 % Savinase 16 L).

### SEQUENCE LISTINGS

SEQ ID NO:1: The amino acid sequence of linker 144K used to attach GH45 catalytic core to CBM.
SEQ ID NO:2: The amino acid sequence of linker 145L used to attach GH45 catalytic core to CBM.
SEQ ID NO:3: The amino acid sequence of linker 146M used to attach GH45 catalytic core to CBM.
SEQ ID NO:4: The full-length amino acid sequence of ACM88 cellulase deriving from the *Acremonium thermophilum* GH45 -cellulase including amino acids from Met1 to Leu298.
SEQ ID NO:5: The nucleotide sequence of the full-length ACM88 cellulase deriving from *Acremonium thermophilum cel45* -cellulase.
SEQ ID NO:6: The amino acid sequence of the full-length DC144 cellulase deriving from the *Acremonium thermophilum* GH45 -cellulase including amino acids from Met1 to Leu298.
SEQ ID NO:7: The nucleotide sequence of the full-length DC144 cellulase deriving from *Acremonium thermophilum cel45* -cellulase.
SEQ ID NO:8: The amino acid sequence of the full-length DC145 cellulase deriving from the *Acremonium thermophilum* GH45 -cellulase including amino acids from Met1 to Leu298.
SEQ ID NO:9: The nucleotide sequence of the full-length DC145 cellulase deriving from *Acremonium thermophilum cel45* -cellulase.
SEQ ID NO:10: The amino acid sequence of the full-length DC146 cellulase deriving from the *Acremonium thermophilum* GH45 -cellulase including amino acids from Met1 to Leu298.
SEQ ID NO:11: The nucleotide sequence of the full-length DC146 cellulase deriving from *Acremonium thermophilum cel45* -cellulase.
SEQ ID NO:12: The full-length amino acid sequence of MA79 cellulase deriving from the *Melanocarpus albomyces* 20K -cellulase including amino acids from Met1 to Ala235.
SEQ ID NO:13: The nucleotide sequence of the full-length MA79 cellulase deriving from *Melanocarpus albomyces* 20K *cel45* -cellulase.
SEQ ID NO:14: The full-length amino acid sequence of MA79+CBM cellulase deriving from the *Melanocarpus albomyces* 20K -cellulase including amino acids from Met1 to Leu304.
SEQ ID NO:15: The nucleotide sequence of the full-length MA79+CBM cellulase deriving from *Melanocarpus albomyces* 20K *cel45* -cellulase.
SEQ ID NO:16: The full-length amino acid sequence of MA79+CBMK cellulase deriving from the *Melanocarpus albomyces* 20K -cellulase including amino acids from Met1 to Leu302.
SEQ ID NO:17: The nucleotide sequence of the full-length MA79+CBMK cellulase deriving from *Melanocarpus albomyces* 20K *cel45* -cellulase.
SEQ ID NO:18: The full-length amino acid sequence of MA79+CBML cellulase deriving from the *Melanocarpus albomyces* 20K -cellulase including amino acids from Met1 to Leu302.
SEQ ID NO:19: The nucleotide sequence of the full-length MA79+CBML cellulase deriving from *Melanocarpus albomyces* 20K *cel45* -cellulase.
SEQ ID NO:20: The full-length amino acid sequence of MA79+CBMM cellulase deriving from the *Melanocarpus albomyces* 20K -cellulase including amino acids from Met1 to Leu302.
SEQ ID NO:21: The nucleotide sequence of the full-length MA79+CBMM cellulase deriving from *Melanocarpus albomyces* 20K *cel45* -cellulase.

### DETAILED DESCRIPTION OF THE INVENTION

A cellulase, or a cellulase component, is an enzyme having cellulolytic activity, which means that it is capable of hydrolysing cellulosic substrates or derivatives thereof into smaller saccharides. Cellulolytic enzymes thus include both cellulases and hemicellulases. Cellulases include (1) endoglucanases (EG, EC 3.2.1.4) which cut internal beta-1,4-glucosidic bonds; (2) exoglucanases or cellobiohydrolases (CBH, EC 3.2.1.176, EC 3.2.1.91) that cut the disaccharide cellobiose from the reducing or non-reducing end of the crystalline cellulose polymer chain and (3) beta-1,4-glucosidases (BG, EC 3.2.1.21) which hydrolyze the cellobiose and other short cello-oligosaccharides to glucose.

The term "fusion cellulase" refers to the fusion protein of the invention.

The present invention relates in particular to endoglucanases as cellulase components. In an embodiment the cellulase component is a fungal endoglucanase belonging to glycosyl hydrolase family 45. "Glycosyl hydrolase family 45" refers to the glycosyl hydrolase family as defined by Henrissat 1991, and Henrissat and Bairoch 1993, 1996.

"Mature polypeptide" means a polypeptide in a form following translation and any post-translational modifications, such as N-terminal processing, C-terminal truncation, glycosylation, and phosphorylation. In an embodiment the cellulase component is a mature polypeptide.

In an embodiment the fusion protein is obtained by recombinant production in a heterologous host cell, preferably a fungal host cell, such as *Trichoderma.*

As used herein, "isolated", "synthetic", and "artificial" mean a substance in a form or environment that does not occur in nature. Non-limiting examples of isolated substances include (1) any non-naturally occurring substance, (2) any substance including any enzyme, variant, nucleic acid, protein, fusion protein, peptide or cofactor, that is at least partially removed from one or more or all of the naturally occurring constituents with which it is associated in nature; (3) any substance modified by the hand of man relative to that substance found in nature, such as a variant; or (4) any substance modified by increasing or decreasing the amount of the substance relative to other components with which it is naturally associated (e.g., recombinant production in a host cell; one or multiple copies of a gene encoding the substance; and use of an alternative promoter to the promoter naturally associated with the gene encoding the substance). In an embodiment a polypeptide, enzyme, variant, polynucleotide, host cell or composition of the invention is isolated.

The term "operably linked", when referring to DNA segments, denotes that the segments are arranged so that they function in concert for their intended purposes, e.g. transcription initiates in the promoter and proceeds through the coding segment to the terminator.

The term "promoter" denotes a portion of a gene containing DNA sequences that provide for the binding of RNA polymerase and initiation of transcription. Promoter sequences are commonly, but not always, found in the 5' non-coding regions of genes.

The term "secretory signal sequence" or "signal sequence" denotes a DNA sequence that encodes a polypeptide (a "secretory peptide") that, as a component of a larger polypeptide, directs the larger polypeptide through a secretory pathway of a host cell in which it is produced. The secretory signal sequence can be native or it can be replaced with secretory signal sequence or carrier sequence from another source. Depending on the host cell, the larger peptide may be cleaved to remove the secretory peptide during transit through the secretory pathway.

The term "core region" or "catalytic domain" or "CD" denotes a domain of an enzyme, which may or may not have been modified or altered, but which has retained at least part of its original activity. The core region of a cellulase component according to the invention corresponds to the amino acids 22-228 of SEQ ID NO: 12 and 18-224 of SEQ ID NO: 4.

In addition to the "catalytic domain", which forms the active or functional site of the enzyme, one or more "cellulose binding domains" ("CBDs"), also named as carbohydrate binding domains/modules (CBD/CBM) may be located either at the N- or C-terminus of the catalytic domain. CBMs have carbohydrate-binding activity and they mediate the binding of cellulase to crystalline and amorphous cellulose, but have little or no effect on hydrolytic activity of the enzyme on soluble substrates. The family 1 CBMs (CBM1s) are found almost exclusively in fungal enzymes. Family 1 modules are usually relatively small, only 35-40 residues long.

An important aspect of the present invention is a linker polypeptide, which was engineered between a catalytic core of the cellulase and the carbohydrate binding domain (carbohydrate binding component) of the "fusion protein" or "fusion cellulase". By the term "linker" is meant a polypeptide between the catalytic core of the cellulase and the carbohydrate binding module (CBM). For example, the fusion protein with a CBM can be manufactured by fusing a DNA sequence encoding the enzyme core, a DNA sequence encoding the linker and a DNA sequence encoding the CBM sequentially into one open reading frame and expressing this construct. The length of the linker sequence may vary between 20-50 amino acids. The linker is typically flexible and susceptible to movement. Preferably the linker has 38 amino acids. In an embodiment the linker region is O-glycosylated. O-glycans present in the linker can confer resistance to proteases and play an important role in the multimodular enzyme structure when engaged on cellulose. Linker glycans may also be directly involved in substrate interactions. Glycans may change the structure and dynamics of the linker peptide. O-glycosylation can be increased by genetic engineering of the linker region.

The following abbreviations are used for amino acids:

| | | |
|---|---|---|
| A | Ala | Alanine |
| C | Cys | Cysteine |
| D | Asp | Aspartic acid |
| E | Glu | Glutamic acid |
| F | Phe | Phenylalanine |
| G | Gly | Glycine |
| H | His | Histidine |
| I | Ile | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |

Substitutions are described using of the following nomenclature: amino acid residue in the protein scaffold; position; substituted amino acid residue(s). According to this nomenclature the substitution of, for instance, a serine residue for a glycine residue at position 20 is indicated as Ser20Gly or S20G.

The term "derived from" or "parental" generally indicates that one specified material has its origin in another specified material or has features that can be described with reference to the other specified material.

As used herein, the term "comprising" includes the broader meanings of "including", "containing", and "comprehending", as well as the narrower expressions "consisting of" and "consisting only of".

As used herein, a "peptide" and a "polypeptide" are amino acid sequences including a plurality of consecutive polymerized amino acid residues. For purpose of this invention, peptides are molecules including up to 19 amino acid residues, and polypeptides include more than 19 amino acid residues. The peptide or polypeptide may include modified amino acid residues, naturally occurring amino acid residues not encoded by a codon, and non-naturally occurring amino acid residues. As used herein, a "protein" may refer to a peptide or a polypeptide of any size. A protein may be an enzyme, a protein, an antibody, a membrane protein, a peptide hormone, regulator, or any other protein.

The term "polynucleotide" denotes a single-stranded or double-stranded polymer of deoxyribonucleotide or ribonucleotide bases read from the 5' to the 3' end. Polynucleotides include RNA and DNA, and may be isolated from natural sources, synthesized in vitro, or prepared from a combination of natural and synthetic molecules.

As used herein, "modification", "modified", and similar terms in the context of polynucleotides refer to modification in a coding or a non-coding region of the polynucleotide, such as a regulatory sequence, 5' untranslated region, 3' untranslated region, up-regulating genetic element, down-regulating genetic element, enhancer, suppressor, promoter, exon, or intron region. The modification may in some embodiments be only structural, having no effect on the biological effect, action or function of the polynucleotide. In other embodiments the modification is a structural modification, which provides a change in the biological effect, action or function of the polynucleotide. Such a modification may enhance, suppress or change the biological function of the polynucleotide.

As used herein, "identity" means the percentage of exact matches of amino acid residues between two aligned sequences over the number of positions where there are residues present in both sequences. When one sequence has a residue with no corresponding residue in the other sequence, a gap is allowed in the alignment. That position is not counted in the denominator of the identity calculation in the alignment program. Identity is a value determined with the Pairwise Sequence Alignment tool EMBOSS Needle at the EMBL-EBI website (www.ebi.ac.uk/Tools/psa/emboss needle/). A higher sequence identity with a polypeptide having enzyme activity may mean more similar functional properties and similar structure of the polypeptide. However, a polypeptide having a lower sequence identity may also have similar properties despite the differences in the primary structure: a polypeptide having low sequence identity may be able to adopt a similar fold and conformation of the critical amino acids e.g. in the substrate binding site, in sites relevant for interaction or conformational changes, and in the active site.

As used herein, "host cell" means any cell type that is susceptible to transformation, transfection, transduction, mating, crossing or the like with a nucleic acid construct or expression vector comprising a polynucleotide. The term "host cell" encompasses any progeny that is not identical due to mutations that occur during replication. Preferably a recombinant host cell is used which is modified to express and secrete the fusion protein of the invention as its main activity or one of its main activities.

As used herein, "expression" includes any step involved in the production of a polypeptide in a host cell including, but not limited to, transcription, translation, post-translational modification, and secretion. Expression may be followed by harvesting, i.e. recovering, the host cells or the expressed product.

The term "expression vector" denotes a DNA molecule, linear or circular, that comprises a segment encoding a polypeptide of interest operably linked to additional segments that provide for its transcription. Such additional segments may include promoter and terminator sequences, and may optionally include one or more origins of replication, one or more selectable markers, an enhancer, a polyadenylation signal, carrier and the like. Expression vector segments can be derived from the host organism, another organism, plasmid or viral DNA, or can be synthetic. The expression vector may be any expression vector that is synthetic or is conveniently subjected to recombinant DNA procedures, and the choice of vector will often depend on the host cell into which the vector is to be introduced. Thus, the vector may be an autonomously replicating vector, i.e. a vector, which exists as an extrachromosomal entity, the replication of which is independent of chromosomal replication, e.g. a plasmid. Alternatively, the vector may be one which, when introduced into a host cell, is integrated into the host cell genome and replicated together with the chromosome(s) into which it has been integrated. In an embodiment the present vector is an expression vector.

The term "recombinant" or "recombinantly produced" used herein in connection with production of a polypeptide or protein is defined according to the standard definition in the art.

The term "obtained from" and "obtainable" as used herein in connection with a specific microbial source of a polynucleotide means that the polynucleotide is expressed by the specific microbial source (homologous expression), or by a cell in which a gene or a polynucleotide from the source has been inserted (heterologous expression).

The term "enzyme preparation" or "enzyme composition" means any enzyme product, preparation or composition, which comprises at least one of the fusion cellulases of the present invention. An enzyme composition may be a spent culture medium or filtrate containing one or more fusion cellulase, or one or more fusion cellulase and one or more other enzymes. "Spent culture medium" means the culture medium of the host comprising the produced enzymes. Preferably the host cells are separated from said medium after the production. The enzyme preparation or composition may be a "whole culture broth" obtained, optionally after inactivating the production host(s) or microorganism(s) without any biomass separation, down-stream processing or purification of the desired cellulolytic enzyme(s), because the present fusion cellulases can be secreted into the culture medium, and they display activity in the ambient conditions of the spent culture medium.

The enzyme composition may contain enzymes in at least partially purified and isolated form. It may even essentially consist of the desired enzyme or enzymes. If desired, the enzyme compositions may be dried, spray-dried or lyophilized, granulated, or the enzymatic activity may be otherwise concentrated and/or stabilized for storage. If required, a desired enzyme may be crystallized or isolated or purified in accordance with conventional methods, such as filtration, extraction, precipitation, chromatography, affinity chromatography, electrophoresis, or the like.

In addition to one or more fusion cellulases of the present invention, the enzyme composition may comprise one or more additional enzymes selected from the group consisting of protease, lipase, cutinase, amylase, carbohydrase, cellulase, pectinase, pectatelyase, pectinolytic enzyme, esterase, phytase, mannanase, arabinase, galactanase, xylanase, oxidase, xanthanase, xyloglucanase, nuclease, laccase, and/or peroxidase, preferably selected from the group consisting of proteases, amylases, cellulases and lipases. More specifically, the enzyme preparation may comprise at least one further enzyme selected from a group of cellobiohydrolase, endoglucanase, beta-glucanase, beta-glucosidase, serine protease, xylanase, beta-xylosidase, mannanase, beta-mannosidase, endopectinlyase, pectate lyase, pectinesterase, laccase, cutinase, peroxidase and copper-dependent lytic polysaccharide monooxygenase i.e. glycosyl hydrolase family 61 (GH61) or Auxiliary Activity family 9 (AA9) enzymes. The enzyme composition may contain any combination of these enzymes and the fusion cellulases of the invention, but the enzymes are not limited to those described herein. The additional enzymes can, for example, also be commercially available enzyme preparations. It depends on the application what other enzymes are included in the enzyme composition or used in the enzyme treatment.

The present enzyme composition comprising cellulase and an additional enzyme may be advantageous in providing synergistic effects. Such additional enzymes are desired when the present enzyme composition comprising cellulase is used in detergents e.g. when washing stains. Particularly advantageous synergistic enzymes that work with cellulases are amylases, proteases and mannanases, or a combination thereof. The perfect combination of enzymes allows maximal performance.

In general the properties of the selected enzyme(s) should be compatible with the selected detergent, (*i.e.,* pH-optimum, compatibility with other enzymatic and non-enzymatic ingredients, etc.), and the enzyme(s) should be present in effective amounts.

The enzyme composition may comprise the fusion cellulase of the invention and:
a. optionally at least one polyol selected from propylene glycol, glycerol, a sugar, sugar alcohol, sorbitol, hexylene glycol;
b. optionally at least one preservative selected preferably from organic acids, e.g. benzoic acid, citric acid, ascorbic acid, sorbic acid, and salts thereof, sodium benzoate, hydroxybenzoate, benzisothiazolinone (BIT) or a combination thereof;
c. optionally at least one inhibitor selected from formic acid, lactic acid, boric acid, boric acid derivative, aromatic borate ester, phenyl boronic acid derivative, peptide, other reversible subtilisin inhibitors or a combination thereof;
d. optionally at least one enzyme selected from proteases, amylases, cellulases, lipases, xylanases, mannanases, cutinases, esterases, phytases, DNAses, pectinases, pectinolytic enzymes, pectate lyases, carbohydrases, arabinases, galactanases, xanthanases, xyloglucanase, laccases, peroxidases and oxidases with or without a mediator, or a combination thereof;
e. optionally at least one salt selected from sodium chloride, potassium chloride, potassium (hydrogen)phosphate, sodium (hydrogen)phosphate, ammonium sulfate, potassium sulfate, or a combination thereof; and
f. optionally at least one filler or carrier selected from maltodextrin, flour, sodium chloride, sulfate, sodium sulfate, sodium acid pyrophosphate, tetrasodium pyrophosphate, polyethylene glycol, or a combination thereof.

The additional components a-f provide improved properties for the present enzyme composition. The enzyme composition is compatible with the additional components and improves applicability of the enzyme composition in various uses. Salts, such as sodium chloride and sodium sulfate function as drying aids.

In an embodiment the present enzyme composition is in the form of a liquid composition or a solid composition such as solution, dispersion, paste, powder, pellet, granule, granulate, coated granulate, tablet, cake, crystal, crystal slurry or gel.

The enzyme composition can be used in cleaning agents or boosters that are added on top of the detergent during or before the wash and that are for example in the form of liquid, gel, powder, granules or tablets. The enzyme composition and detergent components may also be soaked in a carrier like textiles.

In an embodiment the enzyme composition is used in textile and detergent industry, biomass processing and biomass hydrolysis, preferably in biofuel, starch, pulp and paper, food, baking, feed or beverage industries. The invention relates also to a use of the fusion cellulase polypeptides of the invention in detergent applications. The terms "detergent composition" and "detergent" include, unless otherwise indicated, all washing agents in any form such as solid, granular or powder-form, liquid, gel or paste-form, and any combination thereof. The detergent composition may be in the form of a sachet, pouch, tablet or bar, including multicompartment products. The detergent composition can be a free-flowing powder or a liquid. The terms include, unless otherwise stated, all-purpose or heavy-duty washing agents, especially cleaning detergents; liquid fine-fabric, specialty or low-duty detergents; hand dishwashing agents or light duty dishwashing agents, especially those of the high-foaming type; machine dishwashing agents, including the various tablet, granular, liquid and rinseaid types for household and institutional use; liquid cleaning and disinfecting agents, car or carpet shampoos, bathroom cleaners; metal cleaners; as well as cleaning auxiliaries such as bleach additives and "stain-stick" or pre-treat types, and laundry aids.

The terms "detergent", "detergent composition" and "detergent formulation" are used in reference to mixtures, which are intended for use in a wash medium for the cleaning of soiled objects. In some embodiments, the term is used in reference to laundering fabrics and/or garments (e.g., "laundry detergents"). In alternative embodiments, the term refers to other detergents, such as those used to clean dishes, cutlery, etc. (e.g., "dishwashing detergents"). It is not intended that the present invention be limited to any particular detergent formulation or composition. It is intended that in addition to the cellulases according to the invention, the term encompasses detergents that may contain e.g., surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anticorrosion agents, hydrotropes, fabric hueing agents, dispersants, dye transfer inhibiting agents, fluorescent whitening agents, soil release polymers, anti-redepositions agents, anti-shrink agents, anti-wrinkling agents, bactericides, binders, carriers, dyes, enzyme stabilizers, fabric softeners, fillers, foam regulators, perfumes, pigments, buffers, preservatives, sod suppressors, solvents, and structurants for liquid detergents, structure elasticizing agents, enzyme inhibitors or stabilizers, enzyme activators, transferase(s), hydrolytic enzymes, oxido reductases, bluing agents and fluorescent dyes, antioxidants, and solubilizers.

The detergent composition of the invention may comprise one or more components selected from the group consisting of anionic surfactants (0 - 40% by weight), nonionic surfactants (0 - 40% by weight), and phosphonates (0 - 15% by weight) in addition to the effective amount of the fusion cellulase polypeptide or an enzyme preparation thereof.

The term "effective amount" of a cellulase refers to the quantity of the enzyme necessary to perform sufficiently in the specific detergent application. The amount of enzyme preparation in a detergent composition may vary depending on type and concentration of the detergent. Preferably the detergent composition comprises from about 0.000001% to about 10% by weight of the detergent composition of a fusion cellulase of the invention, more preferably from 0.00005% to about 1%, even more preferably from 0.00001 % to 0.1%.

In a preferred embodiment the detergent composition is in the form of liquid detergent or a solid detergent, more preferably in the form of a bar, a homogenous tablet, a tablet having two or more layers, a pouch having one or more compartments, a regular or compact powder, a granule, a paste, a gel, or a regular, compact or concentrated liquid, and
the detergent composition preferably comprises one or more additional enzymes selected from the group consisting of proteases, amylases, cellulases, lipases, xylanases, mannanases, cutinases, esterases, DNAses, pectinases, pectate lyases, pectinolytic enzymes, carbohydrases, arabinases, galactanases, xanthanases, xyloglucanases, laccases, peroxidases and oxidases, preferably from the group of proteases, amylases, cellulases and lipases, and
the detergent composition preferably comprises one or more of the surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anticorrosion agents, hydrotropes, fabric hueing agents, dispersants, dye transfer inhibiting agents, fluorescent whitening agents, soil release polymers, anti-redepositions agents, anti-shrink agents, anti-wrinkling agents, bactericides, binders, carriers, dyes, enzyme stabilizers, fabric softeners, fillers, foam regulators, perfumes, pigments, buffers, preservatives, sod suppressors, solvents, and structurants for liquid detergents, structure elasticizing agents, enzyme inhibitors or stabilizers, enzyme activators, transferase(s), hydrolytic enzymes, oxido reductases, bluing agents and fluorescent dyes, antioxidants, and solubilizers.

The detergent composition may be in the form of a bar, a homogenous tablet, a tablet having two or more layers, a pouch having one or more compartments, a regular or compact powder, a granule, a paste, a gel, or a regular, compact or concentrated liquid. In one embodiment the detergent composition can be a laundry detergent composition, preferably a liquid or solid laundry detergent composition. There are a number of detergent formulation forms such as layers (same or different phases), pouches, as well as forms for machine dosing unit. The present fusion cellulase or the enzyme composition may be added directly into a detergent or it can be applied separately on top of the detergent during or before wash, or, for example, in liquid/liquid or liquid/powder sachets or multicompartment sachets or bottles, in which it may be separated from some of the detergent components or other enzymes, like protease, to maximize the storage stability.

The term "stability" includes storage stability and stability during use, e.g. during a wash process (in wash stability) and reflects the stability of the polypeptide according to the invention as a function of time, e.g. how much activity and/or wash performance is retained when the polypeptide is kept in solution, in particular in a detergent solution. The stability is influenced by many factors, e.g. pH, temperature, detergent composition containing e.g. proteases, stabilizers, builders, surfactants etc. The cellulase stability may be measured using the activity assays or more preferably the application tests described in examples. In a preferred embodiment stability includes the meaning of stability in the presence of proteases.

"Cellulase performance" as used herein means color revival/depilling, antipilling/color care or antigreying effect of cellulases in detergent applications.

As used herein, the term "antigreying performance" or "antigreying effects" mean antiredepositioning and pigment removal properties. With increasing number of wash cycles, pigments, particles and soluble soils, salts and other material can adhere on the textile fibers, most likely in areas with damaged cotton fibers. This can cause a greying effect and a darkening or yellowing of the cotton textile. Suitable test methods are generally known in the art and are typically based on using ballast soil systems with standard white test fabrics in repeated washing cycles in washing machines. The antigreying effect can be tested also by a single wash as stressed test using redeposition liquid based on carbon black. In an embodiment the antigreying effect is determined according to Example 6.

Pilling of fabric may occur in fabrics formed from spun yarns. Pills consist of tangled fibers of the yarn that form on fabric surfaces during wear or laundering. Fabrics with pills have worn appearance and are therefore to be avoided. Pilling has been reduced previously e.g. by using fiber blends or by chemical treatment of the fabric.

The term "depilling" (removal of pilling) means "color revival" capability of a pilled fabric and it can be accomplished by treating the pilled fabric with the fusion protein of the invention. The term "antipilling" (prevention of pilling) means "color maintenance" or "color care" and it can be accomplished by treating the fabric with the fusion protein of the invention. The effect of fiber and color care properties can be detected by visible and measurable decrease of lightness (i.e. increase of darkness) or change in color of colored cotton textiles exposed to repeated washing cycles. Standardized test monitors of prepilled and unpilled, new fabrics are commercially available. In an embodiment the depilling or the antipilling effect is determined as color revival or color care performance according to Example 2. The skilled person is capable of using the teaching of Example 2 to determine an appropriate amount of the fusion protein and an appropriate time needed for the treatment.

The term "textile" means any textile material including yarns, yarn intermediates, fibers, non-woven materials, natural materials, synthetic materials, and any other textile material, fabrics made of these materials and products made from fabrics (e.g., garments, linen and other articles). The textile or fabric may be in the form of knits, wovens, denims, non-wovens, felts, yarns, and towelling. The textile may be cellulose based, such as natural cellulosics including cotton, flax/linen, jute, ramie, sisal or coir or manmade cellulosics (e.g. originating from wood pulp) including viscose/rayon, ramie, cellulose acetate fibers (tricell), lyocell or blends thereof. The textile or fabric may also be non-cellulose based such as natural polyamides including wool, camel, cashmere, mohair, rabbit and silk or synthetic polymer such as nylon, aramid, polyester, acrylic, polypropylen and spandex/elastane, or blends thereof as well as blend of cellulose based and non-cellulose based fibers. Examples of blends are blends of cotton and/or rayon/viscose with one or more companion material such as wool, synthetic fibers (e.g. polyamide fibers, acrylic fibers, polyester fibers, polyvinyl alcohol fibers, polyvinyl chloride fibers, polyurethane fibers, polyurea fibers, aramid fibers), and cellulose-containing fibers (e.g. rayon/viscose, ramie, flax/linen, jute, cellulose acetate fibers, lyocell). Fabric may be conventional washable laundry, for example stained house-hold laundry. When the term fabric or garment is used it is intended to include the broader term textiles as well.

In an embodiment the fusion protein of the invention has a depilling and/or antipilling effect on cotton fabric.

In an embodiment the fusion protein of the invention has a depilling and/or antipilling effect on a fabric containing cellulose, or cellulose derivative.

An advantage of the depilling/antipilling effect of the fusion protein is that it can be easily used to remove pills of e.g. fully cotton fabrics. Further, in fabrics having loose yarn density pilling is typically problematic, and the present fusion protein can be included in detergents intended for laundering such fabrics, thereby preventing pilling.

In an embodiment the fusion protein has improved stability in a liquid detergent in the presence of a protease.

An advantage of the improved stability against proteases is that the fusion protein can be included in various products, such as detergents that typically contain proteases. It is possible to formulate e.g. in liquid detergents with long shelf-life and stability.

In an embodiment the fusion protein has improved stability in liquid detergent containing protease. In an embodiment the stability is determined according to Example 3 or Example 7. In an embodiment the fusion protein has antigreying effect. In an embodiment the antigreying effect is determined according to Example 6.

In an embodiment in the fusion protein of the invention the cellulase component belongs to family GH45 of endoglucanases.

This enzyme family is preferable because of its structural and functional closeness to the enzymes used in the Examples. Thus, because of the similarity the skilled person understands that the cellulase component belonging to this class has the same or similar properties and characteristics with the cellulase components used in the Examples.

In an embodiment the fusion protein comprises an N-terminal cellulase component, a C-terminal carbohydrate binding component, and a linker component between them.

In an embodiment the fusion protein comprises an C-terminal cellulase component, a N-terminal carbohydrate binding component, and a linker component between them. In an embodiment the present fusion protein or the present enzyme composition is for use in detergents, in treating fiber, in wood-derived pulp, in biomass hydrolysis, in food or feed application, or in any application involving modification, degradation or removal of cellulose containing material.

In an embodiment in the fusion protein of the invention the cellulase component has at least 80 % sequence identity over the (core region) residues 18-224 with the SEQ ID NO: 4.

In an embodiment in the fusion protein of the invention the cellulase component has at least 80 % sequence identity over the (core region) residues 22-228 with the SEQ ID NO: 12.

In an embodiment in the fusion protein of the invention the linker component comprises at least 35 amino acids. This is an advantageous length for the linker to allow the cellulase component and the carbohydrate binding component to interact with the substrate and to give the fusion protein of the invention desired properties.

In an embodiment in the fusion protein of the invention the linker component, and according to the numbering corresponding to the SEQ ID NO 1, comprises at least one of the following characteristics:
the amino acid position at position 3 is other than N, preferably S;
the amino acid position at position 8 is other than N, preferably R;
the amino acid position at position 13 is other than N, preferably S; and
the amino acid position at position 18 is other than N, preferably S.

In an embodiment in the fusion protein of the invention the amino acid sequence of the linker component further comprises at least the following characteristics:
the amino acid position at position 12 is other than G, preferably S; and
the amino acid position at position 37 is other than G, preferably T.

In an embodiment the C-terminal amino acid of the cellulase component is Pro, corresponding to the substitution A228P in SEQ ID NO: 14.

In an embodiment the linker component has at least 90% sequence identity with SEQ ID. NO: 1, such as at least 91, 92, 93, 94, 95, 96, 97, 98, or 99% sequence identity. In an embodiment the linker component has the amino acid sequence of SEQ ID NO: 1. These embodiments provide a fusion protein, which has good depilling/antipilling performance and stability in liquid detergent containing protease.

In an embodiment the linker component has at least 88% sequence identity with SEQ ID. NO: 2, such as at least 89, 90, 91, 92, 93, 94, 95, 96, 97, 98, or 99% sequence identity. In an embodiment the linker component has the amino acid sequence of SEQ ID NO: 2. These embodiments provide a fusion protein, which has good depilling/antipilling performance and stability in liquid detergent containing protease.

In an embodiment the linker component has at least 85% sequence identity with SEQ ID: NO: 3, such as at least 86, 87, 88, 89, 90, 91, 92, 93, 94, 95, 96, 97, 98, or 99% sequence identity. In an embodiment the linker component has the amino acid sequence of SEQ ID NO: 3. These embodiments provide a fusion protein, which has good depilling/antipilling performance and stability in liquid detergent containing protease.

In an embodiment in the fusion protein of the invention the carbohydrate binding component belongs to CBM family 1 (CBM1).

In another embodiment the fusion protein of the invention is capable of binding crystalline and amorphous cellulose.

It is advantageous to have a CBD, which binds particularly crystalline or amorphous cellulose because cellulose is typically in this form in fabrics, making it a suitable binding partner for the CBD. Thus, the CBD can bind to the cellulose containing fabric and bring the enzymatically active cellulase component in vicinity of the cellulase substrate, thereby improving the performance of the fusion protein.

In an embodiment in the fusion protein of the invention the carbohydrate binding component has at least 70% sequence identity with the residues 269-304 of SEQ ID NO: 14.

In an embodiment the fusion protein of the invention has at least 90% sequence identity with SEQ ID NO: 14, 16, 18 or 20.

In an embodiment the host cell is preferably selected from the group consisting of: fungal cells,
filamentous fungal cells from Division *Ascomycota,* Subdivision *Pezizomycotina;* preferably from the group consisting of members of the Class *Sordariomycetes,* Subclass *Hypocreomycetidae,* Orders *Hypocreales* and *Microascales* and *Aspergillus, Chrysosporium, Myceliophthora* and *Humicola;*
more preferably from the group consisting of Families *Hypocreacea, Nectriaceae, Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium;*
more preferably from the group consisting of *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Gibberella fujikuroi, G. moniliformis, G. zeaea, Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum, and Aspergillus niger, Aspergillus awamori, Aspergillus oryzae, Chrysosporium lucknowense, Myceliophthora thermophila, Humicola insolens,* and *Humicola grisea,*
bacterial cells, preferably gram positive Bacilli such as *B*. *subtilis, B. licheniformis, B. megaterium, B. amyloliquefaciens, B. pumilus,* gram negative bacteria such as *Escherichia coli,* actinomycetales such as *Streptomyces* sp., and
yeasts, such as *Saccharomyces cerevisiae, Pichia pastoris, Yarrowia lipolytica,* most preferably *Trichoderma reesei* or *Bacillus.*

In an embodiment the enzyme composition of the invention further comprises at least one enzyme selected from proteases, amylases, cellulases, lipases, xylanases, mannanases, cutinases, esterases, phytases, nucleases, pectinases, pectinolytic enzymes, pectate lyases, carbohydrases, arabinases, galactanases, xanthanases, xyloglucanase, laccases, peroxidases and oxidases with or without a mediator, or a combination thereof.

By including in the enzyme composition other enzymes, its properties can be controlled. Because the fusion protein of the present invention is less sensitive to proteolytic degradation, even proteases can be present in the enzyme composition. This is advantageous e.g. in detergent applications, where it is desirable to include enzymes that have activity against various biological materials, to be able to achieve sufficient performance.

In an embodiment in the method of the invention the cellulosic material is textile material, plant material used in animal feed, or wood-derived pulp or secondary fiber.

Any cellulosic material can be degraded by the present fusion protein. Thus, it can be used to modify e.g. rheological properties of feed and pulp.

In an embodiment the method of the invention comprises contacting the textile material with the detergent composition of the seventh aspect.

In an embodiment the method of the invention is laundry, biostoning, biofinishing or antigreying. In these applications the detergent composition of the invention, or the enzyme composition can be used.

In an embodiment the use of the invention is for biofinishing or biostoning textile materials like fabrics or garments or yarn.

Without limiting the scope and interpretation of the patent claims, certain technical effects of one or more of the aspects or embodiments disclosed herein are listed in the following: A technical effect is degradation or modification of cellulose acid. Another technical effect is storage stability of the fusion protein and resistance against proteases.

The foregoing description has provided by way of non-limiting examples of particular implementations and embodiments of the invention a full and informative description of the best mode presently contemplated by the inventors for carrying out the invention. It is however clear to a person skilled in the art that the invention is not restricted to details of the embodiments presented above, but that it can be implemented in other embodiments using equivalent means without deviating from the characteristics of the invention.

Furthermore, some of the features of the above-disclosed aspects and embodiments of this invention may be used to advantage without the corresponding use of other features. As such, the foregoing description should be considered as merely illustrative of the principles of the present invention, and not in limitation thereof. Hence, the scope of the invention is only restricted by the appended patent claims.

In an embodiment at least one component of the compositions of the invention has a different chemical, structural or physical characteristic compared to the corresponding natural component from which the at least one component is derived from. In an embodiment said characteristic is at least one of uniform size, homogeneous dispersion, different isoform, different codon degeneracy, different post-translational modification, different methylation, different tertiary or quaternary structure, different enzyme activity, different affinity, different binding activity, and different immunogenicity.

### EXAMPLES

### Example 1. Production of recombinant cellulase fusion proteins in Trichoderma reesei

Standard molecular biology methods were used in the isolation and enzyme treatments of DNA (e.g. isolation of plasmid DNA, digestion of DNA to produce DNA fragments), in E. coli transformations, sequencing etc. The basic laboratory methods used were either as described by the enzyme, reagent or kit manufacturer or as described in the standard molecular biology handbooks, e.g. Sambrook and Russell (2001) or as described in the following examples.

Cellulase fusion proteins were designed from parental molecule deriving from *Acremonium thermophilum,* designated as ACM88 (WO2016/066896). Variant cellulase ACM88 contains a catalytic core deriving from *A. thermophilum* Cel45A attached to a linker and CBM region deriving from *T. reesei* Cel7A (nucleic acid sequence SEQ ID NO: 5, corresponding to amino acid sequence SEQ ID NO: 4). Expression plasmids were constructed for production of three fusion cellulases, wherein the linker region of ACM88 (SEQ ID NO:4, amino acids 225-262) were changed to linker sequences presented in Table 1. *A. thermophilum* fusion cellulases containing linkers 144K, 145L and 146M, were named DC144, DC145 and DC146, respectively. Synthetic genes DC144 (SEQ ID NO: 6, SEQ ID NO: 7), DC145 (SEQ ID NO: 8, SEQ ID NO: 9) and DC146 (SEQ ID NO: 10, SEQ ID NO: 11) were exactly fused as *Sac*II*-Bam*HI fragments to the *T. reesei cel7*A promoter by ligation. The expression constructs contain *T. reesei cel7*A promoter, *T. reesei cel7*A terminator for transcription termination and the *amdS* marker gene as described in Paloheimo *et al.* 2003 (Figure 1A). A linear expression cassette isolated from the vector backbone by *Eco*RI digestion was used in transformation of *T. reesei* protoplasts and transformants were selected with acetamide as sole nitrogen source. The host strain lacks the four major endogenous cellulases: CBHI/Cel7A, CBHII/Cel6A, EGI/Cel7B and EGII/Cel5A. The transformations were performed according to Penttilä et al, 1987, with the modifications described in Karhunen et al., 1993.

**Table 1. Amino acid sequences (AA) and SEQ ID NO's of synthetic linkers used to attach CBM region to GH45 cellulases.**

| **Synthetic linker** | **Sequence (AA)** | **SEQ ID NO** |
|---|---|---|
| **144K** | VFSPPSGRPSGGSPPGGSPPGTTTTRRPATTTGSSPGP | **1** |
| **145L** | VFSPPSGRPSGGSPPGGSPPGTTTTRRPATTTGSSPTP | **2** |
| **145M** | VFSPPSGRPSGSSPPGGSPPGTTTTRRPATTTGSSPTP | **3** |

Cellulase fusion proteins were designed from parental molecule deriving from *Melanocarpus albomyces* 20K, designated as MA79. Cellulase 20K or variant cellulase MA79, containing mutations G22A and N122A (numbering without signal sequence; mature sequence starting from Ala), do not contain a CBM (nucleic acid sequence SEQ ID NO: 13, corresponding to amino acid sequence SEQ ID NO: 12). Linker and CBM from pALK1770 described in WO2006/117432 was attached to the C-terminus of MA79 catalytic core and the resulting fusion cellulase was named MA79+CBM (SEQ ID NO: 14, SEQ ID NO: 15). The last amino acid prior to linker and CBM attachment was changed from alanine to proline (A228P). To construct fusion cellulase MA79+CBML (SEQ ID NO: 18, SEQ ID NO: 19), the linker region in MA79+CBM (SEQ ID NO: 14, amino acids 229-268) was changed to the linker sequence 145L (Table 1, SEQ ID NO: 2). Synthetic genes MA79+CBM and MA79+CBML were exactly fused as *Sac*II-*Bam*HI fragment to the *T. reesei cel7*A promoter by ligation. The expression constructs contain *T. reesei cel7*A promoter and the *amdS* marker gene as described in Paloheimo *et al.* 2003. *T. reesei* ce/6A terminator was used for transcription termination (Figure 1B). A linear expression cassette isolated from the vector backbone by *Not*I digestion was used in transformation of *T. reesei* protoplasts and transformants were selected with acetamide as sole nitrogen source. The host strain lacks the four major endogenous cellulases: CBHI/Cel7A, CBHII/Cel6A, EGI/Cel7B and EGII/Cel5A. The transformations were performed according to Penttilä *et al,* 1987, with the modifications described in Karhunen *et al.,* 1993.

The endoglucanase production of the transformants was analyzed from the culture supernatants of shake flask cultivations (50 ml). The transformants were grown for 7 days in a complex cellulase-inducing medium (Joutsjoki *et al.,* 1993) buffered with 5% KH₂PO₄ at pH 5.5. The enzyme activity of the recombinant protein was measured from the culture supernatant as the release of reducing sugars from carboxymethylcellulose (3% CMC) at 50°C in 50 mM HEPES buffer, pH 7.0 essentially as described by Bailey. and Nevalainen, 1981; Haakana *et al,* 2004 (NCU activity). Production of the recombinant protein was also detected from the culture supernatant by sodium dodecyl sulfate polyacrylamide gel electrophoresis (SDS-PAGE). Chosen DC144, DC145, DC146 and MA79+CBML transformants and the reference strains producing cellulases ACM88, MA79 and MA79+CBM were cultivated in shake flasks or bioreactors in complex cellulase-inducing medium to obtain material for the application tests (Examples 2 to 4).

To construct fusion cellulases MA79+CBMK (SEQ ID NO: 16, SEQ ID NO: 17) and MA79+CBMM (SEQ ID NO: 20, SEQ ID NO: 21), the linker region in MA79+CBM (SEQ ID NO: 14, amino acids 229-268) is changed to the linker sequence 144K (Table 1, SEQ ID NO: 1) and linker sequence 146M (Table 1, SEQ ID NO: 3), respectively. Synthetic genes MA79+CBMK and MA79+CBMM are exactly fused as *Sac*II*-Bam*HI fragments to the *T. reesei* cel7A promoter for expression and cellulase production in *T. reesei* as described in previous paragraphs. MA79+CBMK and MA79+CBMM show improved stability compared to MA79+CBM measured as described in Examples 3 and 7.

### Example 2. Testing the color revival and color care performance of cellulase fusion proteins MA79+CBM and MA79+CBML in Launder-Ometer

Fusion proteins MA79+CBM and MA79+CBML identified as described in Example 1 were tested for color revival (depilling) and color care (antipilling) performance in a commercial liquid detergent using parental molecule MA79 for comparison.

The following pilling monitors (multicolor printed Jersey, 94% Cotton, 6% Dorlastan) supplied from Center For Testmaterials BV (The Netherlands) were used: E-252 (original fabric, unpilled) and E-253 (prepilled/predamaged material). Monitors E-253 were used for the demonstration of the removal of pilling (depilling) from material representing used cotton textiles. The same predamaged monitors were also used for demonstration of the color revival effect of used colored textiles. Monitors of original fabric (E-252) were used for the demonstration of the color maintenance/color care and/or prevention of pilling (antipilling) effect of new fabrics. Both test fabrics were cut into swatches (approx. 29 cm x 15 - 16.5 cm, total weight of two swatches approx. 24 g) containing full width stripes of each color (black, red, green, blue) and the edges were neatened. The color of unwashed monitors was measured prior to tests to select homogenious material for tests.

Cellulase treatments were performed in Atlas LP-2 Launder-Ometer as follows. Launder-Ometer was first preheated to 40 °C. 60 g of steel balls (diameter 0.6 cm), 1.06 g detergent, 240 ml of synthetic tap water (hardness 16 °dH) and diluted enzyme (<1.0 ml) were added into 1.2 liter containers. After that, one swatch of E-253 and E-252 were placed in containers (reverse side on reverse side) and the Launder-Ometer was run at 40 °C for 60 min with a rotation speed of 42 rpm. Enzymes were dosed 0 - 0.2 activity units (NCU) per ml of wash liquor and (control sample contained no cellulase). Activity was measured as described in Example 1. The wash liquor contained 4.4 g/l of commercial liquid detergent described in Table 2 in Example 3 and its pH was approx. 8.4.

For synthetic tap water with hardness of 16 °dH the following stock solutions were prepared in deionized water (Milli-Q or equivalent):
Stock solution with 1000 °d Calcium-hardness: CaCl₂ x 2 H₂O (1.02382.1000, Merck KGaA, Germany) 26.22 g/l
Stock solution with 200 °d Magnesium-hardness: MgSO₄ x 7 H₂O (1.05886.1000, Merck KGaA, Germany) 8.79 g/l H₂O
NaHCO3 stock solution: NaHCO₃ (1.06329.1000 Merck KGaA, Germany) 29.6 g/l
13.3 ml CaCl₂ solution, 13.3 ml MgSO₄ solution and 10.0 ml of freshly made NaHCO₃ solution were added in volumetric flask in the given order, made up to 1 liter with deionized water and mixed. The hardness of water was determined by complexometric titration and found correct.

After the cellulase treatment in Launder-Ometer, the swatches were first rinsed separately under running water (ca. 20 °C) and then in a washing machine (Whirlpool) using rinsing program with extraction. Swatches were dried in a tumbler. Washing and tumbling cycles were repeated 10 times.

The cellulase performance in detergent was evaluated by measuring the color as reflectance values with Konica Minolta CM-3610A spectrophotometer using L*a*b* color space coordinates (illuminant D65/10°, 420 nm cut). The color of each 4 stripes of test monitors was measured after 10 washing cycles. Decrease of lightness (L*), i.e. increase of darkness compared to treatment without cellulase, was used as an indication of cellulase effect. When the surface fibers and fibrils protruding from the yarn forming pills and giving the fabric a "greyish" or worn out look, are removed by cellulase, the lightness of the fabric decreases, and the surface of the fabric appears darker and colors get brighter. The color was measured from the swatches also before the cellulase treatment to select homogenous material for the tests.

Cellulase performance was calculated as ΔL* (delta L*), which means lightness value L* of enzyme treated fabric minus lightness value L* of fabric treated with washing liquor without cellulase enzyme (enzyme blank, control). Sum of ΔL* for each 4 stripes was calculated and the final results were shown as increase of darkness (-ΔL*).

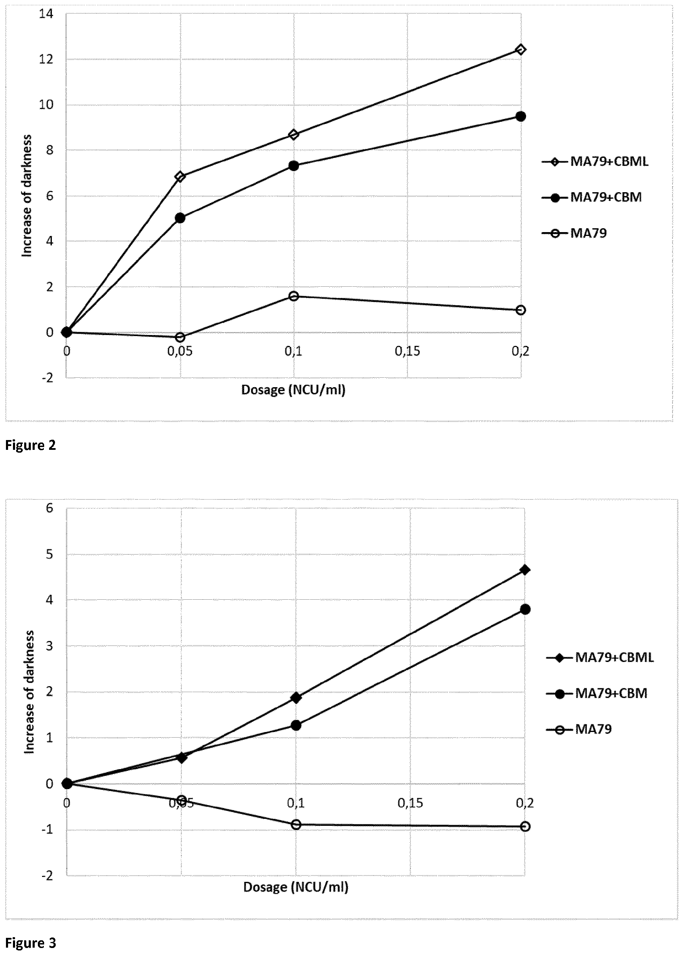

Color revival (depilling) effect of cellulase fusion proteins MA79+CBM and MA79+CBML is shown in Figure 2 and color care (antipilling) effect in Figure 3. Parental molecule MA79 was used for comparison. Parental molecule without CBM did not show improvement in color care properties and had no significant depilling effect. Fusion cellulases MA79+CBM and MA79+CBML had high depilling and antipilling effect. MA79+CBML was even more effective in color and fiber care than MA79+CBM.

### Example 3. Testing the stability of fusion proteins MA79+CBM and MA79+CBML in detergent at room temperature measured as color revival (depilling) performance

The stability of fusion cellulases MA79+CBM and MA79+CBML was tested by application tests as color revival (depilling effect) using parental MA79 for comparison and modified test system from that described in Example 2. Enzyme dosage was increased from 0.2 to 2 NCU/ml per wash liquor and therefore amount of washing cycles could be reduced to 3 instead of 10 to obtain a faster and less laborious test system. Two pieces of prepilled test fabrics (E-253) were used as test material in each Launder-Ometer container. Enzyme preparations with similar activity levels, prepared from bioreactor cultivation samples produced as described in Example 1, were used in tests.

A 0.7 % w/w amount of protease, Savinase 16L (Novozymes), was added to a commercial liquid detergent containing no enzymes. The composition of detergent is described in Table 2. Cellulases were initially added in detergent in such amount that dosages would be 2 activity units (NCU) per ml of wash solution, when detergent was used 4,4 g/l. Activity was measured as described in Example 1 except using extended reaction time of 30 min. Samples in plastic tubes with caps were incubated at room temperature (approx. 20 - 22 °C) for 4 days. The performance of stored samples was compared to washes, in which the cellulases had been added fresh into washing liquor containing detergent and same amount of protease that was initially added to the stored samples, corresponding to initial performance at timepoint 0. Results were calculated as residual performance (%) which was obtained by dividing the performance of sample after storage by the initial performance (fresh performance) of the sample.

**Table 2. Composition of commercial liquid detergent**

| **Ingredient** | **%** |
|---|---|
| Anionic surfactants | 15 - 30 |
| Nonionic surfactants, soap | 5 - 15 |
| Phosphonate, Soap | < 5 |
| Boric acid | ≤1 |
| Other ingredients: e.g. optical brighteners, perfumes, preservatives | |
| pH 8.2-8.6 | |

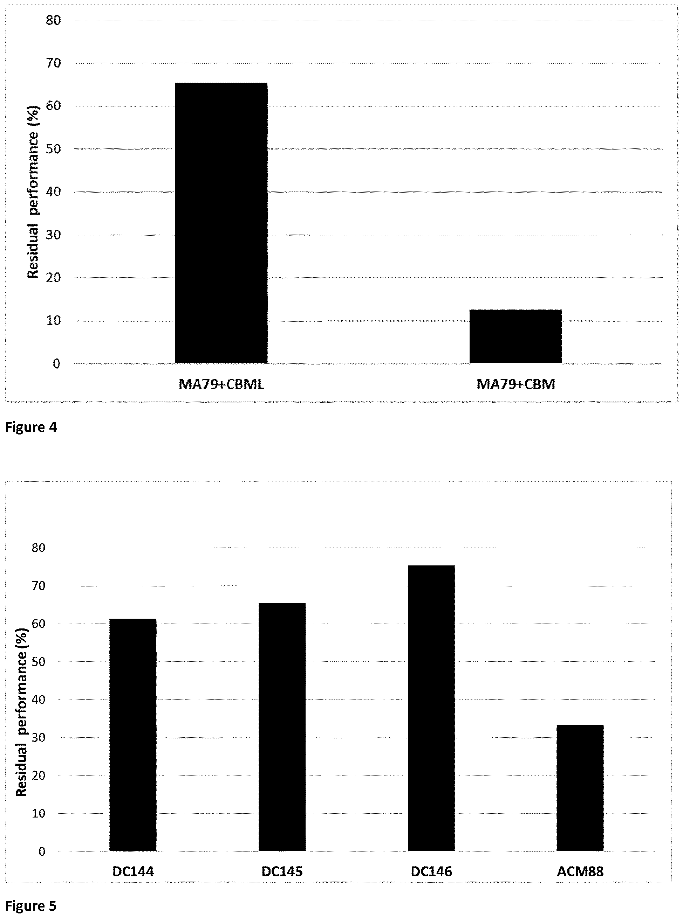

The stability of fusion cellulases MA79+CBM and MA79+CBML is shown in Figure 4 as residual performance. Parental molecule MA79 could not be used as a reference, since it had no significant depilling effect. MA79+CBML had considerably better performance compared to MA79+CBM after 4 days storage in a protease containing detergent at room temperature. The stability of MA79+CBML measured as application performance was about 5 times better than that with MA79+CBM.

The stability of cellulases was measured also by analyzing the NCU activity. Results were calculated as residual activity (%) which was obtained by dividing the activity of sample after storage by the initial activity of the sample. However, the stability measured as enzyme activity showed no significant loss in activity or differences between samples: residual activity of M79, MA79+CBM and MA79+CBML was ≥96 %. Analytical activity based on soluble substrate did not correlate with the stability measured as depilling effect with the cellulose binding module containing molecules, especially when unstable linker (CBM) was used.

### Example 4. Testing the color revival performance and its stability in detergent at room temperature with fusion proteins DC144 - DC146

Enzyme preparations with similar activity levels, prepared from bioreactor cultivation samples of of fusion cellulases DC144, DC145 and DC146 produced in *Trichoderma* and identified as described in Example 1, were tested for color revival (depilling) performance in a commercial liquid detergent using ACM88 for comparison. The performance was measured also after 4 days storage at room temperature in detergent in the presence of protease. Test system was similar than that described in Example 3.

The stability of fusion cellulases DC144 - DC146 compared to ACM88 is shown in Figure 5 as residual color revival performance. DC144, DC145 and DC146 had considerably better performance compared ACM88 after 4 days storage in a protease containing detergent (0.7 % Savinase 16L) at room temperature. The stability of fusion cellulases measured as application performance was at least 1.8 times better compared to parental ACM88 in liquid detergent containing protease.

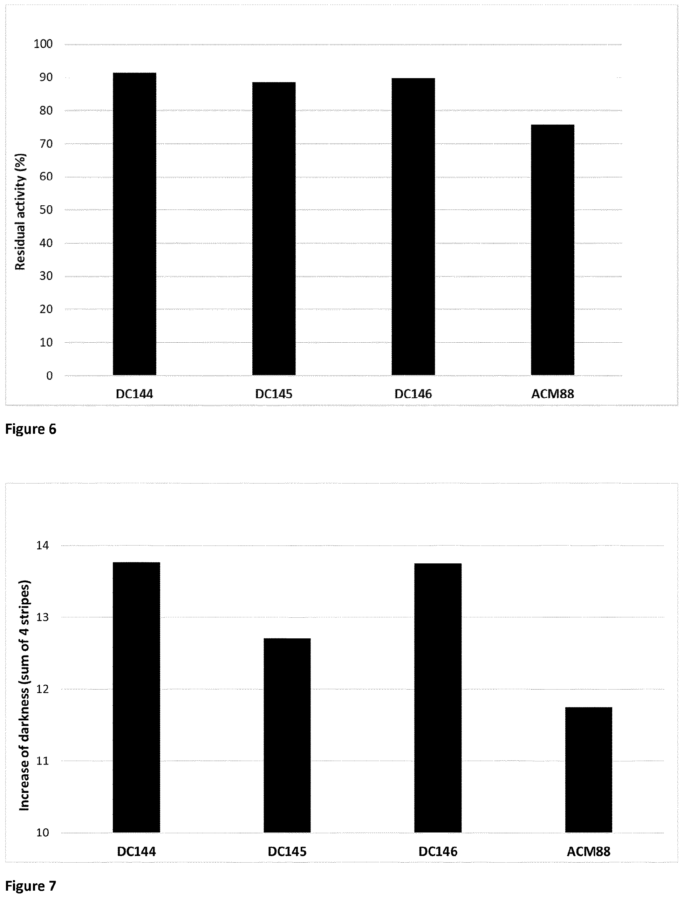

The stability of fusion cellulases was measured also by analyzing the NCU activity. Results were calculated as residual activity (%) which was obtained by dividing the activity of sample after storage by the initial activity of the sample. The loss of performance was much greater than the loss of the analytical NCU-activity on CMC-substrate after storage in protease containing detergent, as shown in Figure 6. This was found to be mainly a result from loss of CBD which was detected by SDS-PAGE analysis (data not shown). Loss of CBD affects more on the performance than on the analytical activity, therefore the stability of fusion proteins measured with NCU activity assay showed considerably smaller differences compared to ACM88. However, the stability of fusion cellulases DC144-DC146 was increased compared to ACM88 also when measured as residual activity.

Also, the fresh performance of DC144, DC145 and DC146 was better compared to ACM88, when the color revival effect was measured after 3 washing and tumbling cycles and using enzyme dosage of 2 NCU/g (Figure 7).

### Example 5. Testing the stability of fusion proteins as color revival performance in a commercial detergent concentrate at 30 °C

Enzyme preparations with similar activity levels, prepared from bioreactor cultivation samples of fusion cellulases DC144, DC145, DC146 and MA79+CBML and the references ACM88 and MA79+CBM identified as described in Example 1, were tested for color revival (depilling) performance in a commercial detergent using a test system similar to that described in Example 3, except having different detergent and different storage conditions.

The performance was measured after 11 days storage at 30 °C in commercial detergent concentrate containing a commercial protease without cellulase. Cellulases were initially added in detergent in such amount that dosages would be 2 activity units (NCU) per ml of wash solution. The wash liquor contained 3.2 g/l of detergent concentrate and its pH was approx. 8.5. Results were calculated as residual performance as described in Example 3.

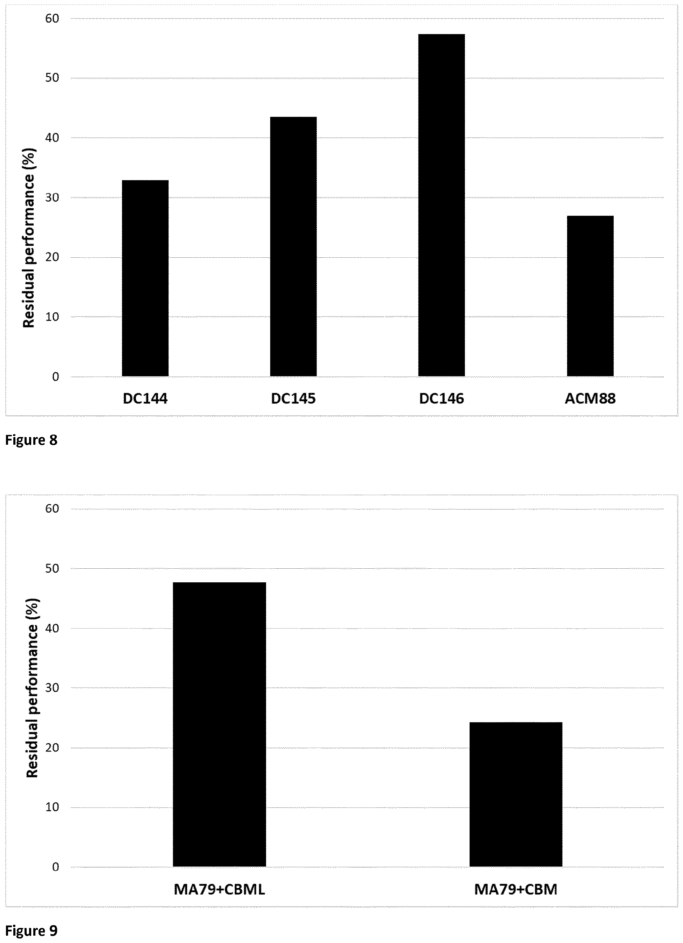

All fusion cellulases DC144, DC145 and especially DC146 showed considerably better stability compared to ACM88 as described in Figure 8 as residual color revival performance. Also fusion protein MA79+CBML had remarkably better stability of performance compared to MA79+CBM (Figure 9). Results show that fusion proteins having a stable linker had improved stability of color revival performance when stored several days in a protease containing detergent even at elevated temperatures like 30 °C.

### Example 6. Testing the antigreying performance of fusion proteins in Launder-Ometer

Enzyme preparations with similar activity levels, prepared from bioreactor cultivation samples of fusion cellulases MA79+CBM and MA79+CBML were tested for anti-greying performance by a single wash method (40 °C, 60 min, 16 °dH) using carbon black (approx. 0.15 g/l) in a wash solution in addition to detergent (4.4 g/l). Parental cellulase MA79 known to be efficient in antigreying was used as reference.

Cotton interlock double jersey with optical brighteners (CN-42) supplied from CFT (Center for Testmaterials NV, the Netherlands) was used as test fabric. The fabric was first prewashed in a washing machine (15 min 50 °C and 60 min at 60 °C) and tumble dried, then cutted to swatches of approx. 14-14.5 cm (total weight of 4 swatches 25 g).

As a source of carbon black RD-liq 01 from CFT containing 7 g of carbon black liquid (about 33 % carbon black) in plastic bottles, that are normally intended for full scale washes in washing machine (one bottle per one single wash with test fabrics), were used. In this Example the method was adapted to small scale using approximately similar ratio of carbon black and water that would be in full scale. First a stock solution carbon black was prepared by placing an opened bottle of RD-liq 01 (i.e. about 2.3 g carbon black) in a decanter flask containing 1 liter of deionized water. The solution was stirred with a magnetic stirrer for overnight until the contents of the bottle were totally released. After that 65 g of stock solution was mixed with 935 ml of synthetic tap water with hardness of 17.1 °dH ending up to diluted carbon black solution having hardness of 16 °dH and carbon black content approximately 0.15 g/l (or 0.45 g RD-liq 01).

For synthetic tap water with hardness of 17.1 °dH the following stock solutions were prepared in deionized water (Milli-Q or equivalent):
Stock solution with 1000°d Calcium-hardness: CaCl₂ x 2 H₂O (1.02382.1000, Merck KGaA, Germany) 26.22 g/l
Stock solution with 200°d Magnesium-hardness: MgSO₄ x 7 H₂O (1.05886.1000, Merck KGaA, Germany) 8.79 g/l H₂O
NaHCO₃ stock solution: NaHCO₃ (1.06329.1000 Merck KGaA, Germany) 29.6 g/l.

14.2 ml CaCl₂ solution, 14.2 ml MgSO₄ solution and 10.0 ml of freshly made NaHCO₃ solution were added in volumetric flask in the given order, made up to 1 liter with deionized water and mixed. The hardness of water was deter-mined by complexometric titration and found correct.

Antigreying tests were performed in Atlas LP-2 Launder-Ometer as follows. Launder-Ometer was first preheated to 40 °C. 60 g of steel balls (diameter 0.6 cm), 1.1 g of commercial liquid detergent described in Example 2, 250 ml of diluted carbon black liquor and diluted enzyme (<1.0 ml) were added into 1.2 liter containers. After that, 4 swatches of prewashed test fabric CN-42 were added and the Launder-Ometer was run at 40 °C for 60 min with a rotation speed of 42 rpm. Enzymes were dosed 0, 0.5, 0.1 and 0.2 activity units (NCU) per liter. Control sample contained no enzyme. Activity was measured as described in Example 1.

After the cellulase treatment in Launder-Ometer, the swatches were first quickly rinsed separately under running tap water (ca. 20 °C) to remove the steel balls, then rinsed separately under running water in specific cups containing holes for 3 times and finally dipped in a bucket containing water. After that the swatches were extracted in a washing machine and let to dry on a grid at room temperature. Enzyme treated fabrics and controls without enzyme were rinsed and extracted separately to avoid contamination.

Antigreying performance of cellulase was evaluated by measuring reflectance of test fabrics by Konica Minolta CM3610A spectrophotometer as Y-value (illuminant D65/10°, 420 nm cut). Cellulase performance was calculated as ΔY (delta Y), which means value Y of enzyme treated fabric minus value Y of fabric treated with carbon black and detergent containing washing liquor without enzyme (enzyme blank, control). Values were the average of 4 swatches. The higher the Y or ΔY value, the better the antigreying effect and whiteness of the fabric.

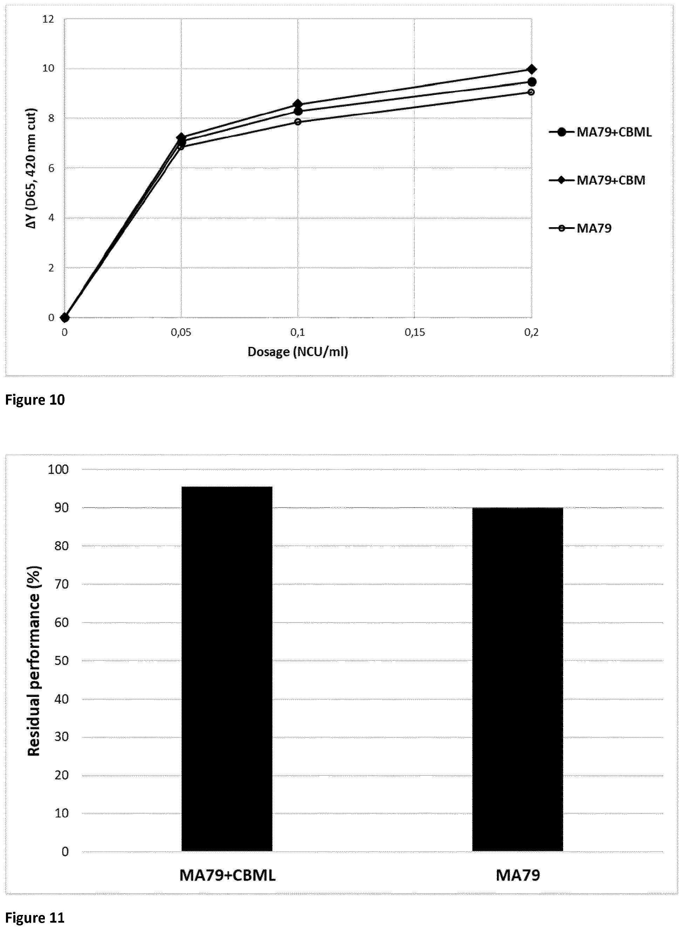

Results in Figure 10 show fusion protein MA79+CBML had excellent antigreying properties, at least as good as parental MA79 cellulase in liquid detergent. Also fusion proteins of DC145 and DC146 had excellent antigreying properties, at least as good as the reference ACM88 in liquid detergent (data not shown).

### Example 7. Testing the stability of fusion protein MA79+CBML in commercial detergent at 37 °C as antigreying performance

The stability of fusion protein MA79+CBML compared to the parental MA79 cellulase was evaluated as antigreying performance. Enzyme preparations with similar activity levels were prepared from bioreactor cultivation samples produced as described in Example 1. Antigreying tests similar to that described in Example 6 were carried out with detergent samples stored at 37 °C. A 0.8% w/w amount of protease, Savinase 16L (Novozymes), was added to a commercial liquid detergent containing no enzymes. The composition of detergent is described in Table 2. Cellulases were initially added to the detergent in such amount that dosage would be 0.2 activity units (NCU) per liter of wash solution. Activity was measured as described in Example 1 except using extended reaction time of 30 min. Samples in plastic tubes with caps were incubated for 11 days at 37 °C. Performance of stored samples was compared to washes, in which the cellulases had been added fresh into washing liquor containing detergent and same amount of protease than the stored samples. Commercial liquid detergent was used 4.4 g/l wash liquor. Results were calculated as residual performance (%) as described in Example 3.

Results in Figure 11 show that fusion protein MA79+CBML had excellent antigreying performance, at least as good as with MA79, after several days storage in a protease containing detergent at high temperature like 37 °C. Based on the results shown in Examples fusion protein MA79 +CBML having improved stability and excellent properties in depilling and antipilling and antigreying application, is beneficial for combi products in which both antigreying and color revival/color care effects are desired.

### REFERENCES

Bailey M and Nevalainen H. 1981. Induction, isolation and testing of stable Trichoderma reesei mutants with improved production of solubilizing cellulase. Enzyme Microb. Technol. 3:153-157.
Haakana H, Miettinen-Oinonen A, Joutsjoki V, Mäntylä A, Suominen P and Vehmaanperä J. 2004. Cloning of cellulase genes from Melanocarpus albomyces and their efficient expression in Trichoderma reesei. Enzyme Microb. Technol. 34:159-167.
Henrissat B. 1991. A classification of glycosyl hydrolases based on amino acid sequence similarities. Biochem. J. 280: 309-316.
Henrissat B. and Bairoch A. 1993. New families in the classification of glycosyl hydrolases based on amino acid sequence similarities. Bio-chem. J. 293: 781-788. Henrissat B. and Bairoch A. 1996. Updating the sequence-based classification of glycosyl hydrolases. Biochem. J. 316: 695-696.
Joutsjoki VV, TK Torkkeli and KMH Nevalainen. 1993. Transformation of Trichoderma reesei with the Hormoconis resinae glucoamylase P (gamP) gene: production of a heterologous glucoamylase by Trichoderma reesei. Curr. Genet. 24:223-228.
Karhunen T, A Mäntylä, KMH Nevalainen and PL Suominen. 1993. High frequency one-step gene replacement in Trichoderma reesei. I. Endoglucanase I overproduction. Mol. Gen. Genet. 241:515-522.
Paloheimo M, A Mäntylä, J Kallio, and P Suominen. 2003. High-yield production of a bacterial xylanase in the filamentous fungus Trichoderma reesei requires a carrier polypeptide with an intact domain structure. Appl. Env. Microbiol. 69:7073-7082.
Penttilä M, H Nevalainen, M Rättö, E Salminen and J Knowles. 1987. A versatile transformation system for the cellulolytic filamentous fungus Trichoderma reesei. Gene 61:155-164.
Sambrook J and Russell DW. 2001. Molecular cloning, a laboratory manual. Cold Spring Harbor Laboratory, New York, US.

## Claims

1. A fusion protein comprising a cellulase component, a linker component, and a carbohydrate binding component, wherein:
the cellulase component is an endoglucanase;
the linker component has at least 90% sequence identity with SEQ ID NO: 1, 2 or 3; and
the carbohydrate binding component has capability to bind cellulose.

2. The fusion protein of claim 1 having a depilling and/or antipilling effect on fabric containing cellulose or cellulose derivative.

3. The fusion protein of claims 1-2 having an improved stability in a liquid detergent in the presence of a protease.

4. The fusion protein of claims 1-3 wherein the cellulase component belongs to family GH45.

5. The fusion protein of claim 1-4 wherein the cellulase component has at least 80% sequence identity over the residues 18-224 with the SEQ ID NO: 4.

6. The fusion protein of claims 1-5 wherein the cellulase component has at least 80% sequence identity over the residues 22-228 with the SEQ ID NO: 12.

7. The fusion protein of claims 1-6, wherein the linker component comprises at least 35 amino acids.

8. The fusion protein of claims 1-7, wherein the amino acid sequence of the linker component, and according to the numbering corresponding to the SEQ ID NO 1, comprises at least one of the following characteristics:
the amino acid position at position 3 is other than N, preferably S;
the amino acid position at position 8 is other than N, preferably R;
the amino acid position at position 13 is other than N, preferably S; and
the amino acid position at position 18 is other than N, preferably S.

9. The fusion protein of claim 8, wherein the amino acid sequence of the linker component further comprises at least the following characteristics:
the amino acid position at position 12 is other than G, preferably S; and
the amino acid position at position 37 is other than G, preferably T.

10. The fusion protein of claims 1-9 wherein the carbohydrate binding component belongs to CBM family 1 (CBM1).

11. The fusion protein of claims 1-10 wherein the carbohydrate binding component has at least 70% sequence identity with the residues 269-304 of SEQ ID NO: 14.

12. The fusion protein of claims 1-11 having at least 90% sequence identity with SEQ ID NO: 14, 16, 18 or 20.

13. An isolated nucleic acid encoding the fusion protein of claims 1-12.

14. A recombinant expression vector comprising the isolated nucleic acid of claim 13 operably linked to regulatory sequences capable of directing expression of a gene encoding said fusion protein in a host.

15. A host cell comprising the fusion protein of claims 1-12, or the recombinant expression vector of claims 14, the host cell preferably being selected from the group consisting of:
fungal cells,
filamentous fungal cells from Division *Ascomycota,* Subdivision *Pezizomycotina;*
preferably from the group consisting of members of the Class *Sordariomycetes,* Subclass *Hypocreomycetidae,* Orders *Hypocreales* and *Microascales* and *Aspergillus, Chrysosporium, Myceliophthora* and *Humicola;*
more preferably from the group consisting of Families *Hypocreacea, Nectriaceae, Clavicipitaceae, Microascaceae,* and Genera *Trichoderma* (anamorph of *Hypocrea), Fusarium, Gibberella, Nectria, Stachybotrys, Claviceps, Metarhizium, Villosiclava, Ophiocordyceps, Cephalosporium,* and *Scedosporium;*
more preferably from the group consisting of *Trichoderma reesei (Hypocrea jecorina), T. citrinoviridae, T. longibrachiatum, T. virens, T. harzianum, T. asperellum, T. atroviridae, T. parareesei, Fusarium oxysporum, F. gramineanum, F. pseudograminearum, F. venenatum, Gibberella fujikuroi, G. moniliformis, G. zeaea, Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum,* and *Scedosporium apiospermum, and Aspergillus niger, Aspergillus awamori, Aspergillus oryzae, Chrysosporium lucknowense, Myceliophthora thermophila, Humicola insolens,* and *Humicola grisea,*
bacterial cells, preferably gram-positive Bacilli such as *B*. *subtilis, B. licheniformis, B. megaterium, B. amyloliquefaciens, B. pumilus,* gram negative bacteria such as *Escherichia coli,* actinomycetales such as *Streptomyces* sp., and
yeasts, such as *Saccharomyces cerevisiae, Pichia pastoris, Yarrowia lipolytica,* most preferably *Trichoderma reesei* or *Bacillus.*

16. A method of producing the fusion protein of any one of claims 1-12 comprising the steps of transforming a host cell with an expression vector encoding said fusion protein, culturing said host cell under conditions enabling expression of said fusion protein, and optionally recovering and purifying said fusion protein.

17. An enzyme composition comprising the fusion protein of any one of claims 1-12, and
optionally at least one polyol selected from propylene glycol, glycerol, a sugar, sugar alcohol, sorbitol, hexylene glycol;
optionally at least one preservative selected for example from organic acids, e.g. benzoic acid, citric acid, ascorbic acid, sorbic acid, and salts thereof, sodium benzoate, hydroxybenzoate, benzisothiazolinone (BIT) or a combination thereof;
optionally at least one inhibitor selected from formic acid, lactic acid, boric acid, boric acid derivative, aromatic borate ester, phenyl boronic acid derivative, peptide, other reversible subtilisin inhibitors or a combination thereof;
optionally at least one enzyme selected from proteases, amylases, cellulases, lipases, xylanases, mannanases, cutinases, esterases, phytases, nucleases, pectinases, pectinolytic enzymes, pectate lyases, carbohydrases, arabinases, galactanases, xanthanases, xyloglucanase, laccases, peroxidases and oxidases with or without a mediator, or a combination thereof;
optionally at least one salt selected from sodium chloride, potassium chloride, potassium (hydrogen)phosphate, sodium (hydrogen)phosphate, ammonium sulfate, potassium sulfate, or a combination thereof; and
optionally at least one filler or carrier selected from maltodextrin, flour, sodium chloride, sulfate, sodium sulfate, sodium acid pyrophosphate, tetrasodium pyrophosphate, polyethylene glycol, or a combination thereof.

18. The enzyme composition according to claim 17, **characterized in that** said enzyme composition is in the form of liquid composition or a solid composition such as solution, dispersion, paste, powder, granule, granulate, coated granulate, tablet, cake, crystal, crystal slurry, gel, or pellet.

19. A detergent composition comprising the fusion protein of any one of claims 1-12 or an enzyme composition of claims 17-18, and
wherein the detergent composition is preferably in the form of liquid detergent or a solid detergent, more preferably in the form of a bar, a homogenous tablet, a tablet having two or more layers, a pouch having one or more compartments, a regular or compact powder, a granule, a paste, a gel, or a regular, compact or concentrated liquid, and
wherein the detergent composition preferably comprises one or more additional enzymes selected from the group consisting of proteases, amylases, cellulases, lipases, xylanases, mannanases, cutinases, esterases, DNAses, pectinases, pectate lyases, pectinolytic enzymes, carbohydrases, arabinases, galactanases, xanthanases, xyloglucanases, laccases, peroxidases and oxidases, preferably from the group of proteases, amylases, cellulases and lipases, and
wherein the detergent composition preferably comprises one or more of the surfactants, builders, chelators or chelating agents, bleach system or bleach components, polymers, fabric conditioners, foam boosters, suds suppressors, dyes, perfume, tannish inhibitors, optical brighteners, bactericides, fungicides, soil suspending agents, anticorrosion agents, hydrotropes, fabric hueing agents, dispersants, dye transfer inhibiting agents, fluorescent whitening agents, soil release polymers, anti-redepositions agents, anti-shrink agents, anti-wrinkling agents, bactericides, binders, carriers, dyes, enzyme stabilizers, fabric softeners, fillers, foam regulators, perfumes, pigments, buffers, preservatives, sod suppressors, solvents, and structurants for liquid detergents, structure elasticizing agents, enzyme inhibitors or stabilizers, enzyme activators, transferase(s), hydrolytic enzymes, oxido reductases, bluing agents and fluorescent dyes, antioxidants, and solubilizers.

20. A method for treating cellulosic material, wherein the method comprises reacting the cellulosic material with the fusion protein of any one of claims 1-12 or the enzyme composition of claims 17-18.

21. The method of claim 20, wherein the cellulosic material is textile material, plant material used in animal feed, or wood-derived pulp or secondary fiber.

22. The method of claim 21, wherein the method comprises contacting the textile material with the detergent composition of claim 17-19.

23. A method for antigreying, stain removal, fiber and color care, biostoning or biofinishing which comprises a step of adding the fusion protein of any one of claims 1 to 12 or the enzyme composition according to claims 17-18 to liquid used in treating fabric containing cellulose or cellulose derivative or garments or other textile materials like fabrics or garments or yarn.

24. The method of claim 23, wherein the textile materials are manufactured of natural cellulose containing fibers or manmade cellulose containing fibers or mixtures thereof.

25. Use of the fusion protein of any one of claims 1-12, or the enzyme composition according to claims 17-18 in detergents, in treating fiber, in wood-derived pulp, in biomass hydrolysis, in textile application, in food or feed application, or in any application involving modification, degradation or removal of cellulose containing material.
